# EUROPÄISCHE PATENTSCHRIFT

(11) **EP 0 967 208 B1**
(45) Veröffentlichungstag und Bekanntmachung des Hinweises auf die Patenterteilung: **14.08.2002**
(21) Anmeldenummer: 99105429.7
(22) Anmeldetag: 17.03.1999
(51) Int. Cl.: C07D 239/54, C08K 5/3462

(54) **In 5-Stellung substituierte 6-Aminouracile als Stabilisatoren für halogen-haltige Polymere**
In 5-position substituted 6-aminouracils as stabilisers for halogen-containing polymers
6-aminouracils substitués en position 5 comme stabilisants pour des polymères halogenes

(30) Priorität: 26.06.1998 CH 137198
(43) Veröffentlichungstag der Anmeldung: 29.12.1999
(73) Patentinhaber: Crompton Vinyl Additives GmbH, 68623 Lampertheim (DE)
(72) Erfinder: Wehner, Wolfgang, Dr., 64673 Zwingenberg (DE); Friedrich, Hans-Helmut, 64686 Lautertal-Gadernheim (DE)

(56) Entgegenhaltungen:
- EP-A- 0 065 934
- JAEWON L. SHIM ET AL.: "STUDIES ON THE ACYLATION OF S. 6-AMINOURACIL DERIVATIVES." JOURNAL OF ORGANIC CHEMISTRY., Bd. 37, Nr. 4, 1972, Seiten 578-581, XP002116751 AMERICAN CHEMICAL SOCIETY. EASTON., US ISSN: 0022-3263
- J.-L. BERNIER ET AL.: "NO 116.SUBSTITUTION EN 5 SUR LE DIMETHYL-1,3 AMINO-4 URACILE." BULLETIN DE LA SOCIETE CHIMIQUE DE FRANCE. 2 PARTIE - CHIMIE ORGANIQUE, BIOCHIMIE., Bd. 3-4, Nr. 2, 1976, Seiten 616-620, XP002116752 SOCIETE FRANCAISE DE CHIMIE. PARIS., FR
- KOSAKU HIROTA ET AL.: "THE DIMROTH REARRANGEMENT OF 6-AMINO URACIL DERIVATIVES." CHEMICAL AND PHARMACEUTICAL BULLETIN., Bd. 40, Nr. 10, Oktober 1992 (1992-10), Seiten 2839-2841, XP002116753 PHARMACEUTICAL SOCIETY OF JAPAN. TOKYO., JP ISSN: 0009-2363

## Beschreibung

Die Erfindung betrifft in 5-Stellung substituierte 6-Aminouracile der unten dargestellten Formel I zum Stabilisieren chlorhaltiger Polymere, insbesondere PVC.

PVC kann durch eine Reihe von Zusatzstoffen stabilisiert werden. Verbindungen des Bleis, Bariums und Cadmiums sind dafür besonders gut geeignet, sind jedoch heute aus ökologischen Gründen oder wegen ihres Schwermetallgehalts umstritten
(vgl. "Kunststoffadditive", R. Gächter/H. Müller, Carl Hanser Verlag, 3. Aufl., 1989,
Seiten 303-311, und "Kunststoff Handbuch PVC", Band 2/1, W. Becker/D. Braun,
Carl Hanser Verlag, 2. Aufl., 1985, Seiten 531 - 538; sowie Kirk-Othmer: "Encyclopedia of Chemical Technology", 4^{th} Ed., 1994, Vol. 12, Heat Stabilizers, S. 1071 1091). Man sucht daher weiter nach wirksamen Stabilisatoren und Stabilisatorkombinationen, welche frei von Blei, Barium und Cadmium sind.

1,3-disubstituierte Aminouracile sind bereits in US 3,436,362, US 4,656,209, US 4,352,903 und EP-A-0 768 336 beschrieben worden, und können nach bekannten Methoden in einem (oder mehreren) Verfahrensschritt(en) hergestellt werden.

Ebenso sind substituierte Aminouracile bekannt durch die Veröffentlichungen in Jaewon L. Shim et al, J. of Organic Chemistry, Band 37, Nr. 4 (1972), 578-581; J.L. Bernier et al in Bulletin de la Societé Chemique de France 2. Partie Band 3-4, Nr. 2 (1976), 616-620 und aus Kosaku Hirota et al in Chemical and Pharmaceutical Bulletin Band 40, Nr. 10 (1992) 2839-2841. Die letztgenannten Literaturstellen behandeln die pharmakologische Anwendung substituierter Aminouracile.

Es wurde nun gefunden, dass die in 5-Stellung substituierten 6-Aminouracile der allgemeinen Formel II 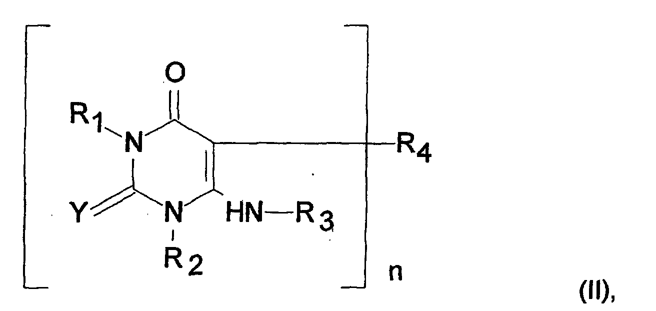 wobei
- n: 1 oder 2 ist,
- Y: Sauerstoff oder Schwefel ist,
- R₁ und R₂: unabhängig voneinander unsubstituiertes oder mit C₁-C₄-Alkoxy-, C₅-C₈-Cycloalkyl-, -OH und/oder Cl substituiertes C₁-C₁₈-Alkyl, C₃-C₆-Alkenyl, C₅-C₈-Cycloalkyl, unsubstituiertes oder am Phenylring mit C₁-C₄-Alkyl-, C₁-C₄-Alkoxy-, C₅-C₈-Cycloalkyl-, -OH und/oder Cl substituiertes C₇-C₉-Phenylalkyl oder Phenyl darstellen,
- R₃: H, unsubstituiertes oder mit -OH, substituiertes C₁-C₁₈-Alkyl oder unsubstituiertes oder mit -OH substituiertes Phenyl darstellt, wenn n=1 ist,
- R₄: -(C=O)-C₁-C₁₂-Alkyl, -(C=O)-O-C₁-C₁₂-Alkyl, -(C=O)-Cₒ-C₁₂-Alkylen-(C=O)OZ,
wobei Z= H oder C₁-C₆-Alkyl ist, -(C=O)-Phenyl, oder 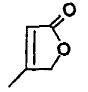 bedeutet,
und, wenn
- n: 2 ist,
- R₄: die Gruppe -CHR₅- bedeutet, wobei
- R₅: H, C₁-C₁₂-Alkyl, unsubstituiertes oder mit -OH, C₁-C₄-Alkyl, C₁-C₄-Alkoxy-, -C=O(OR₆) und/oder -O-COR₆ substituiertes Phenyl bedeutet und
- R₆: verzweigtes oder unverzweigtes C₁-C₁₂-Alkyl oder verzweigtes oder unverzweigtes C₂-C₁₂-Alkenyl ist
sich besonders gut für die Stabilisierung von chlorhaltigen Polymeren wie z. B. PVC eignen.

Für Verbindungen der Formel II gilt:
C₁-C₄-Alkyl bedeutet z. B. Methyl, Ethyl, n-Propyl, iso-Propyl, n-, i-, sec- oder t-Butyl.
C₅-C₁₈-Alkyl bedeutet z. B. Hexyl, Heptyl, Octyl, 2-Ethylhexyl, i-Octyl, Decyl, Nonyl, Undecyl, Dodecyl, Pentadecyl, Hexadecyl u.s.w.
Bevorzugt sind Methyl, Ethyl, Propyl und Butyl, insbesondere Methyl und n-Butyl.

C₃-C₆-Alkenyl bedeutet Allyl, 1-Butenyl, 2-Butenyl, 3-Butenyl, Isobutenyl, 1-Pentenyl, 2-Pentenyl, 3-Pentenyl, 4-Pentenyl, 1-Hexenyl, 2-Hexenyl, 3-Hexenyl, 4-Hexenyl, 5-Hexenyl als auch deren Isomeren. Bevorzugt ist Allyl.
C₁-C₄-Alkoxy bedeutet z. B. Methoxy, Ethoxy, n-Propoxy und n-Butoxy als auch Isopropoxy, Isobutoxy und tert.-Butoxy.
C₅-C₈-Cycloalkyl bedeutet z. B. Cyclopentyl, Cyclohexyl, Cycloheptyl oder Cyclooctyl, bevorzugt Cyclohexyl.
Bei C₇-C₁₀-Phenylalkyl handelt es sich beispielsweise um Benzyl, 1- oder 2-Phenylethyl, 3-Phenylpropyl, α,α-Dimethylbenzyl oder 2-Phenylisopropyl, vorzugsweise um Benzyl und 2-Phenethyl, insbesondere um Benzyl.
Wenn der aromatische Rest substituiert ist, dann bevorzugt mit drei, zwei oder insbesondere einem Substituenten, und die Substituenten sind vor allem Hydroxy, Chlor, Methyl, Ethyl, tert.-Butyl, Methoxy oder Ethoxy. Ganz besonders bevorzugt sind Hydroxy, Methoxy und tert.-Butyl.
Cₒ-C₁₂-Alkylen bedeutet entweder eine direkte Bindung oder beispielsweise Methylen, Ethylen, Propylen, Butylen, Octylen oder Dodecenylen als auch alle möglichen Isomeren davon.

Bevorzugt sind Verbindungen der allgemeinen Formel I 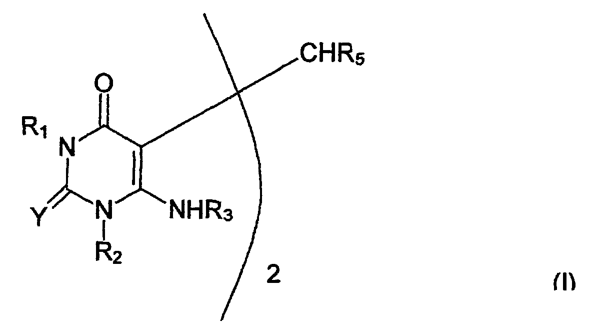 wobei
- Y: Sauerstoff oder Schwefel ist,
- R₁ und R₂: unabhängig voneinander unsubstituiertes oder mit C₁-C₄-Alkoxy-, C₅-C₈-Cycloalkyl-, -OH und/oder Cl substituiertes C₁-C₁₈-Alkyl, C₃-C₆-Alkenyl, C₅-C₈-Cycloalkyl, unsubstituiertes oder am Phenylring mit C₁-C₄-Alkyl-, C₁-C₄-Alkoxy-, C₅-C₈-Cycloalkyl-, -OH und/oder Cl substituiertes C₇-C₉-Phenylalkyl oder Phenyl darstellen,
- R₃: H, unsubstituiertes oder mit -OH substituiertes C₁-C₁₈-Alkyl oder unsubstituiertes oder mit -OH substituiertes Phenyl ist, und
- R₅: H, C₁-C₁₂-Alkyl, unsubstituiertes oder mit -OH, C₁-C₄-Alkyl, C₁-C₄-Alkoxy-, -C=O(OR₆) und/oder -O-COR₆ substituiertes Phenyl bedeutet und
- R₆: verzweigtes oder unverzweigtes C₁-C₁₂-Alkyl oder verzweigtes oder unverzweigtes C₂-C₁₂-Alkenyl ist.

Weiterhin bevorzugt sind Verbindungen der Formel I, worin Y Sauerstoff bedeutet.

Ebenso bevorzugt sind Verbindungen der allgemeinen Formel I, wobei
- Y: Sauerstoff und
- R₁ und R₂: unabhängig voneinander C₁-C₈-Alkyl, Allyl oder C₇-C₉-Phenylalkyl darstellen, und
- R₅: H, C₁-C₈-Alkyl, unsubstituiertes oder mit -OH, C₁-C₄-Alkyl- und/oder C₁-C₄-Alkoxy- substituiertes Phenyl bedeutet.

Die Verbindungen der Formel I sind zur Erzielung der Stabilisierung im chlorhaltigen Polymer zweckmäßig zu 0,01 bis 10 Gew.-%, vorzugsweise zu 0,05 bis 5 Gew.-%, insbesondere zu 0,1 bis 3 Gew.-% zu verwenden.

Weiterhin können Kombinationenen von Verbindungen der allgemeinen Formel I mit anderen üblichen Additiven bzw. Stabilisatoren eingesetzt werden, beispielsweise mit Polyolen und Disaccharidalkoholen und/oder Perchloratverbindungen und/oder Glycidylverbindungen und/oder zeolithischen Verbindungen und/oder Schichtgitterverbindungen (Hydrotalcite) sowie z. B. Lichtschutzmittel.
Beispiele für solche zusätzlichen Komponenten sind nachfolgend aufgeführt und erläutert.

### Polyole und Disaccharidalkohole

Als Verbindungen dieses Typs kommen beispielsweise in Betracht: Pentaerythrit, Dipentaerythrit, Tripentaerythrit, Trimethylolethan, Bistrimethylolpropan, Inosit (Cyclite), Polyvinylalkohol, Bistrimethylolethan, Trimethylolpropan, Sorbit (Hexite), Maltit, Isomaltit, Cellobiit, Lactit, Lycasin, Mannit, Lactose, Leucrose, Tris-(hydroxyethyl)-isocyanurat, Tris-(hydroxypropyl)-isocyanurat, Palatinit, Tetramethylolcyclohexanol, Tetramethylolcyclopentanol, Tetramethylolcyclopyranol, Xylit, Arabinit (Pentite), Tetrite, Glycerin, Diglycerin, Polyglycerin, Thiodiglycerin oder 1-0-a-D-Glycopyranosyl-D-mannit-dihydrat. Bevorzugt sind davon die Disaccharidalkohole.
Verwendung finden können auch Polyolsirupe, wie Sorbit-, Mannit- und Maltitsirup.
Die Polyole können in einer Menge von beispielsweise 0,01 bis 20, zweckmäßig von
0,1 bis 20 und insbesondere von 0,1 bis 10 Gew.-Teilen bezogen auf 100 Gew.-Teile PVC, angewandt werden.

### Perchlorat-Verbindungen

Beispiele sind diejenigen der Formel M(ClO₄)ₙ, wobei M für Li, Na, K, Mg, Ca, Sr, Ba, Zn, Al, La oder Ce steht. Der Index n ist entsprechend der Wertigkeit von M 1, 2 oder 3. Die Perchloratsalze können mit Alkoholen (Polyolen,Cyclodextrinen), oder Ätheralkoholen bzw. Esteralkoholen komplexiert oder gelöst sein. Zu den Esteralkoholen sind auch die Polyolpartialester zu zählen. Bei mehrwertigen Alkoholen oder Polyolen kommen auch deren Dimere, Trimere, Oligomere und Polymere in Frage, wie Di-, Tri-, Tetra- und Polyglycole, sowie Di-, Tri- und Tetrapentaerythrit oder Polyvinylalkohol in verschiedenen Polymerisationsgraden.
Als weitere Lösungsmittel kommen Phosphatester sowie cyclische und acyclische Kohlensäureester in Frage.
Die Perchloratsalze können dabei in verschiedenen gängigen Darreichungsformen eingesetzt werden; z. B als Salz oder Lösung in Wasser oder einem organischen Solvens als solches, bzw. aufgezogen auf ein Trägermaterial wie PVC, Ca-Silikat, Zeolithe oder Hydrotalcite, oder eingebunden durch chemische Reaktion in einen Hydrotalcit oder eine andere Schichtgitterverbindung. Als Polyolpartialether sind Glycerinmonoether und Glycerinmonothioether bevorzugt.

Weitere Ausführungsformen werden beschrieben in EP 0 394 547, EP 0 457 471 und WO 94/24200.
Die Perchlorate können in einer Menge von beispielsweise 0,001 bis 5, zweckmäßig 0,01 bis 3, besonders bevorzugt 0,01 bis 2 Gew.-Teilen, bezogen auf 100 Gew.-Teile PVC, angewandt werden.

### Glycidylverbindungen

Sie enthalten die Glycidylgruppe 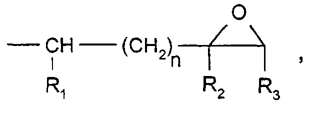 wobei diese direkt an Kohlenstoff, Sauerstoff-, Stickstoff- oder Schwefelatome gebunden ist, und worin entweder R₁ und R₃ beide Wasserstoff sind, R₂ Wasserstoff oder Methyl und n = 0 ist, oder worin R₁ und R₃ zusammen -CH₂-CH₂- oder -CH₂-CH₂-CH₂- bedeuten, R₂ dann Wasserstoff und
n = 0 oder 1 ist.
I) Glycidyl- und β-Methylglycidylester erhältlich durch Umsetzung einer Verbindung mit mindestens einer Carboxylgruppe im Molekül und Epichlorhydrin bzw. Glycerindichlorhydrin bzw. β-Methyl-epichlorhydrin. Die Umsetzung erfolgt zweckmäßig in Gegenwart von Basen.
   Als Verbindungen mit mindestens einer Carboxylgruppe im Molekül können aliphatische Carbonsäuren verwandt werden. Beispiele für diese Carbonsäuren sind Glutarsäure, Adipinsäure, Pimelinsäure, Korksäure, Azelainsäure, Sebazinsäure oder dimerisierte bzw. trimerisierte Linolsäure, Acryl- und Methacrylsäure, Capron-, Capryl-, Laurin-, Myristin-, Palmitin-, Stearin- und Pelargonsäure, sowie die bei den organischen Zinkverbindungen erwähnten Säuren.
   Es können aber auch cycloaliphatische Carbonsäuren eingesetzt werden, wie beispielsweise Cyclohexancarbonsäure, Tetrahydrophthalsäure, 4-Methyltetrahydrophthalsäure, Hexahydrophthalsäure oder 4-Methylhexahydrophthalsäure. Weiterhin können aromatische Carbonsäuren Verwendung finden, wie beispielsweise Benzoesäure, Phthalsäure, Isophthalsäure, Trimellithsäure oder Pyromellithsäure.
   Ebenfalls können auch carboxylterminierte Addukte, z. B. von Trimellithsäure und Polyolen, wie beispielsweise Glycerin oder 2,2-Bis-(4-hydroxycyclohexyl)-propan verwandt werden.
   Weitere im Rahmen dieser Erfindung verwendbare Epoxidverbindungen finden sich in der EP 0506 617.
II) Glycidyl- oder (β-Methylglycidyl)-ether erhältlich durch Umsetzung einer Verbindung mit mindestens einer freien alkoholischen Hydroxygruppe und/oder phenolischen Hydroxygruppe und einem geeignet substituierten Epichlorhydrin unter alkalischen Bedingungen, oder in Anwesenheit eines sauren Katalysators und anschließender Alkalibehandlung.
   Ether dieses Typs leiten sich beispielsweise ab von acyclischen Alkoholen, wie Ethylenglykol, Diethylenglykol und höheren Poly-(oxyethylen)-glykolen, Propan-1,2-diol, oder Poly-(oxypropylen)-glykolen, Propan-1,3-diol, Butan-1,4-diol, Poly-(oxytetramethylen)-glykolen, Pentan-1,5-diol, Hexan-1,6-diol, Hexan-2,4,6-triol, Glycerin, 1,1,1-Trimethylolpropan, Bistrimethylolpropan, Pentaerythrit, Sorbit, sowie von Polyepichlorhydrinen, Butanol, Amylalkohol, Pentanol, sowie von monofunktionellen Alkoholen wie Isooctanol, 2-Ethylhexanol, Isodecanol sowie C₇-C₉-Alkanol- und C₉-C₁₁-Alkanolgemischen.
   Sie leiten sich aber auch beispielsweise ab von cycloaliphatischen Alkoholen wie 1,3- oder 1,4-Dihydroxycyclohexan, Bis-(4-hydroxycyclohexyl)-methan, 2,2-Bis-(4-hydroxycyclohexyl)-propan oder 1,1-Bis-(hydroxymethyl)-cyclohex-3-en oder sie besitzen aromatische Kerne wie N,N-Bis-(2-hydroxyethyl)-anilin oder p,p'-Bis-(2-hydroxyethylamino)-diphenylmethan.
   Die Epoxidverbindungen können sich auch von einkernigen Phenolen ableiten, wie beispielsweise von Phenol, Resorcin oder Hydrochinon; oder sie basieren auf mehrkernigen Phenolen wie beispielsweise auf Bis-(4-hydroxyphenyl)-methan, 2,2-Bis-(4-hydroxyphenyl)-propan, 2,2-Bis-(3,5-dibrom-4-hydroxyphenyl)-propan, 4,4'-Dihydroxydiphenylsulfon oder auf unter sauren Bedingungen erhaltene Kondensationsprodukte von Phenolen mit Formaldehyd wie Phenol-Novolake.
   Weitere mögliche endständige Epoxide sind beispielsweise: Glycidyl-1-naphthylether, Glycidyl-2-phenylphenylether, 2-Biphenylglycidylether, N-(2,3-epoxypropyl)-phthalimid und 2,3-Epoxypropyl-4-methoxyphenylether.
III) (N-Glycidyl)-Verbindungen erhältlich durch Dehydrochlorierung der Reaktionsprodukte von Epichlorhydrin mit Aminen, die mindestens ein Aminowasserstoffatom enthalten. Bei diesen Aminen handelt es sich zum Beispiel um Anilin, N-Methylanilin, Toluidin, n-Butylamin, Bis-(4-aminophenyl)-methan, m-Xylylendiamin oder Bis-(4-methylaminophenyl)-methan, aber auch N,N,O-Triglycidyl-m-aminophenol oder N,N,O-Triglycidyl-p-aminophenol.
   Zu den (N-Glycidyl)-Verbindungen zählen aber auch N,N'-Di-, N,N',N"-Tri- und N,N',N",N"'-Tetraglycidylderivate von Cycloalkylenharnstoffen, wie Ethylenharnstoff oder 1,3-Propylenharnstoff, und N,N'-Diglycidylderivate von Hydantoinen, wie von 5,5-Dimethylhydantoin oder Glykoluril und Triglycidylisocyanurat.
IV) S-Glycidyl-Verbindungen, wie beispielsweise Di-S-glycidylderivate, die sich von Dithiolen, wie beispielsweise Ethan-1,2-dithiol oder Bis-(4-mercaptomethylphenyl)-ether ableiten.
V) Epoxidverbindungen mit einem Rest der obigen Formel, worin R₁ und R₃ zusammen -CH₂-CH₂- bedeuten und n 0 ist, sind Bis-(2,3-epoxycyclopentyl)-ether, 2,3-Epoxycyclopentylglycidylether oder 1,2-Bis-(2,3-epoxycyclopentyloxy)-ethan. Ein Epoxidharz mit einem Rest der obigen Formel, worin R₁ und R₃ zusammen -CH₂-CH₂- sind und n 1 bedeutet, ist beispielsweise 3,4-Epoxy-6-methyl-cyclohexancarbonsäure-(3',4'-epoxy-6'-methyl-cyclohexyl)-methylester.

Geeignete endständige Epoxide sind beispielsweise:
a) flüssige Bisphenol-A-diglycidylether wie Araldit®GY 240, Araldit®GY 250, Araldit®GY 260, Araldit®GY 266, Araldit®GY 2600, Araldit®MY 790;
b) feste Bisphenol-A-diglycidylether wie Araldit®GT 6071, Araldit®GT 7071, Araldit®GT 7072, Araldit®GT 6063, Araldit®GT 7203, Araldit®GT 6064, Araldit®GT 7304, Araldit®GT 7004, Araldit®GT 6084, Araldit®GT 1999, Araldit®GT 7077, Araldit®GT 6097, Araldit®GT 7097, Araldit®GT 7008, Araldit®GT 6099, Araldit®GT 6608, Araldit®GT 6609, Araldit®GT 6610;
c) flüssige Bisphenol-F-diglycidylether wie Araldit®GY 281, Araldit®PY 302, Araldit®PY 306;
d) feste Polyglycidylether von Tetraphenylethan wie CG Epoxy Resin®0163;
e) feste und flüssige Polyglycidylether von Phenolformaldehyd Novolak wie EPN 1138, EPN 1139, GY 1180, PY 307;
f) feste und flüssige Polyglycidylether von o-Cresolformaldehyd Novolak wie ECN 1235, ECN 1273, ECN 1280, ECN 1299;
g) flüssige Glycidylether von Alkoholen wie Shell® Glycidylether 162, Araldit®DY 0390, Araldit®DY 0391;
h) flüssige Glycidylether von Carbonsäuren wie Shell®Cardura E Terephthalsäureester, Tri mellithsäureester, Araldit®PY 284;
i) feste heterocyclische Epoxidharze (Triglycidylisocyanurat) wie Araldit® PT 810;
j) flüssige cycloaliphatische Epoxidharze wie Araldit®CY 179;
k) flüssige N,N,O-Triglycidylether von p-Aminophenol wie Araldit®MY 0510;
l) Tetraglycidyl-4-4'-methylenbenzamin oder N,N,N',N'-Tetraglycidyldiaminophenylmethan wie Araldit®MY 720, Araldit®MY 721.

Vorzugsweise finden Epoxidverbindungen mit zwei funktionellen Gruppen Verwendung. Es können aber auch prinzipiell Epoxidverbindungen mit einer, drei oder mehr funktionellen Gruppen eingesetzt werden.

Vorwiegend werden Epoxidverbindungen, vor allem Diglycidylverbindungen, mit aromatischen Gruppen eingesetzt.
Gegebenenfalls kann auch ein Gemisch verschiedener Epoxidverbindungen eingesetzt werden.
Besonders bevorzugt sind als endständige Epoxidverbindungen Diglycidylether auf der Basis von Bisphenolen, wie beispielsweise von 2,2-Bis-(4-hydroxyphenyl)-propan (Bisphenol-A), Bis-(4-hydroxyphenyl)-methan oder Mischungen von Bis-(ortho/para-hydroxyphenyl)-methan (Bisphenol-F).
Die endständigen Epoxidverbindungen können in einer Menge von vorzugsweise mindestens 0,1 Teil, beispielsweise 0,1 bis 50, zweckmäßig 1 bis 30 und insbesondere 1 bis 25 Gew.-Teilen, bezogen auf 100 Gew.-Teile PVC, eingesetzt werden.

### Hydrotalcite

Die chemische Zusammensetzung dieser Verbindungen ist dem Fachmann bekannt,
z. B. aus den Patentschriften DE 3 843 581, US 4,000,100, EP 0 062 813 und WO 93/20135.
Verbindungen aus der Reihe der Hydrotalcite können durch die folgende allgemeine Formel

M²⁺ ₁₋ₓ M³⁺ ₓ(OH)₂ (A^{b-})_{x/b} • d H₂O

beschrieben werden,
wobei
- M²⁺ =: eines oder mehrere der Metalle aus der Gruppe Mg, Ca, Sr, Zn oder Sn ist,
- M³⁺ =: Al, oder B ist,
- Aⁿ: ein Anion mit der Valenz n darstellt,
b eine Zahl von 1 - 2 ist,
0 < x < 0,5 ist,
- m: eine Zahl von 0 - 20 ist.
Bevorzugt ist
- Aⁿ =: OH⁻, ClO₄⁻, HCO₃⁻, CH₃COO⁻, C₆H₅COO⁻, CO₃²⁻, (CHOHCOO)₂²⁻, (CH₂COO)₂²⁻, CH₃CHOHCOO⁻, HPO₃⁻oder HPO₄²⁻
darstellt;
Beispiele für Hydrotalcite sind
Al₂O₃.6MgO.CO₂.12H₂O (i), Mg_{4,5}Al₂(OH)₁₃.CO₃.3,5H₂O (ii), 4MgO.Al₂O₃.CO₂.9H₂O (iii), 4MgO.Al₂O₃.CO₂.6H₂O,
ZnO.3MgO.Al₂O₃.CO₂.8-9H₂O -9H₂O und ZnO.3MgO.Al₂O₃.CO₂.5-6H₂O.
Ganz besonders bevorzugt sind die Typen i, ii und iii.

### Zeolithe (Alkali bzw. Erdalkalialumosilikate)

Sie können durch die folgende allgemeine Formel

M_{x/n}[(AlO₂)ₓ(SiO₂)_{y}].wH₂O

beschrieben werden,
worin n die Ladung des Kations M;
M ein Element der ersten oder zweiten Hauptgruppe, wie Li, Na, K, Mg, Ca, Sr oder Ba;
y : x eine Zahl von 0,8 bis 15, bevorzugt von 0,8 bis 1,2; und
w eine Zahl von 0 bis 300, bevorzugt von 0,5 bis 30, ist.
Beispiele für Zeolithe sind Natriumalumosilikate der Formeln
Na₁₂Al₁₂Si₁₂O₄₈ . 27 H₂O [Zeolith A], Na₆Al₆Si₆O₂₄ . 2 NaX . 7,5 H₂O, X= OH, Halogen, ClO₄ [Sodalith]; Na₆Al₆Si₃₀O₇₂ . 24 H₂O; Na₈Al₈Si₄₀O₉₆ . 24 H₂O; Na₁₆Al₁₆Si₂₄O₈₀ . 16 H₂O; Na₁₆Al₁₆Si₃₂O₉₆ . 16 H₂O; Na₅₆Al₅₆Si₁₃₆O₃₈₄ . 250 H₂O [Zeolith Y], Na₈₆Al₈₆Si₁₀₆O₃₈₄ . 264 H₂O [Zeolith X];
oder die durch teilweisen bzw. vollständigen Austausch der Na-Atome durch Li-, K-, Mg-, Ca-, Sr- oder Zn-Atome darstellbaren Zeolithe wie (Na,K)₁₀Al₁₀Si₂₂O₆₄ . 20 H₂O ; Ca_{4,5}Na₃[(AlO₂)₁₂(SiO₂)₁₂] . 30 H₂O; K₉Na₃[(AlO₂)₁₂(SiO₂)₁₂]. 27 *H*_{*2*}*O*,
Bevorzugte Zeolithe entsprechen den Formeln

Na₁₂Al₁₂Si₁₂O₄₈ . 27 H₂O [Zeolith A],

Na₆Al₆Si₆O₂₄ . 2NaX . 7,5 H₂O, X = OH, Cl, ClO₄, 1/2CO₃ [Sodalith]

Na₆Al₆Si₃₀O₇₂ . 24 H₂O,

Na₈Al₈Si₄₀O₉₆ . 24 H₂O,

Na₁₆Al₁₆Si₂₄O₈₀ . 16 H₂O,

Na₁₆Al₁₆Si₃₂O₉₆ . 16 H₂O,

Na₅₆Al₅₆Si₁₃₆O₃₈₄ . 250 H₂O [Zeolith Y],

Na₈₆Al₈₆Si₁₀₆O₃₈₄ . 264 H₂O [Zeolith X]

und solche X- und Y-Zeolithe mit einem Al/ Si-Verhältnis von ca. 1:1.
oder die durch teilweisen bzw. vollständigen Austausch der Na-Atome durch Li-, K-, Mg-, Ca-, Sr-, Ba- oder Zn-Atome darstellbaren Zeolithe wie

(Na,K)₁₀Al₁₀Si₂₂O₆₄.20 H₂O.

Ca_{4,5}Na₃[(AlO₂)₁₂(SiO₂)₁₂].30 H₂O

K₉Na₃[(AlO₂)₁₂(SiO₂)₁₂] . 27 H₂O

Die angeführten Zeolithe können auch wasserärmer bzw. wasserfrei sein. Weitere geeigente Zeolithe sind:

Na₂O·Al₂O₃·(2 bis 5) SiO₂·(3,5 bis 10) H₂O [Zeolith P]

Na₂O·Al₂O₃·2 SiO₂·(3.5-10)H₂O (Zeolith MAP)

oder die durch teilweisen bzw. vollständigen Austausch der Na-Atome durch Li-, K- oder H-Atome darstellbaren Zeolithe wie

(Li,Na,K,H)₁₀AI₁₀Si₂₂O₆₄ . 20 H₂O.

K₉Na₃[(AlO₂)₁₂(SiO₂)₁₂] . 27 H₂O

K₄Al₄Si₄O₁₆·6H₂O [Zeolith K-F]

Na₈Al₈Si₄₀O₉₆.24 H₂O [Zeolith D],

wie in Barrer et al., J. Chem. Soc. **1952**, 1561 - 71, und in US 2,950,952 beschrieben;
Ferner kommen folgende Zeolithe in Frage:
K-Offretit, wie in EP-A-400,961 beschrieben;
Zeolith R, wie in GB 841,812 beschrieben;
Zeolith LZ-217, wie in US 4,503,023 beschrieben;
Ca-freier Zeolith LZ-218, wie in US 4,333,859 beschrieben;
Zeolith T, Zeolith LZ-220, wie in US 4,503,023 beschrieben;
Na₃K₆Al₉Si₂₇O₇₂.21 H₂O [Zeolith L];
Zeolith LZ-211, wie in US 4,503,023 beschrieben;
Zeolith LZ-212, wie in US 4,503,023 beschrieben;
Zeolith O, Zeolith LZ-217, wie in US 4,503,023 beschrieben;
Zeolith LZ-219, wie in US 4,503,023 beschrieben;
Zeolith Rho, Zeolith LZ-214, wie in US 4,503,023 beschrieben;
Zeolith ZK-19, wie in Am. Mineral. **54** 1607 (1969) beschrieben;
Zeolith W (K-M), wie in Barrer et al., J. Chem. Soc. **1956**, 2882, beschrieben;
Na₃₀Al₃₀Si₆₆O₁₉₂. 98 H₂O [Zeolith ZK-5, Zeolith Q]
Besonders bevorzugt werden Zeolith P-Typen der obigen Formel verwandt,
worin x 2 bis 5 und y 3.5 bis 10 sind, ganz besonders bevorzugt Zeolith MAP der genannten Formel, worin x 2 und y 3.5 bis 10 sind. Insbesondere handelt es sich um Zeolith Na-P, d. h. M steht für Na. Dieser Zeolith tritt im allgemeinen in den Varianten Na-P-1, NaP-2 und Na-P-3 auf, die sich durch ihre kubische, tetragonale oder orthorhombische Struktur unterscheiden (R. M. Barrer, B. M. Munday, J.Chem.Soc. A **1971,** 2909 - 14). In der ebengenannten Literatur ist auch die Herstellung von Zeolith P-1 und P-2 beschrieben. Zeolith P-3 ist danach sehr selten und daher kaum von praktischem Interesse. Die Struktur des Zeolithen P-1 entspricht der aus dem obengenannten Atlas of Zeolite Structures bekannten Gismonditstruktur. In neuerer Literatur
(EP-A-384 070) wird zwischen kubischem (Zeolith B oder P_{C}) und tetragonalem (Zeolith P₁) Zeolithen vom P-Typ unterschieden. Dort werden auch neuere Zeolithen des P-Typs mit Si:Al Verhältnissen unter 1,07:1 genannt. Hierbei handelt es sich um Zeolithe mit der Bezeichnung MAP oder MA-P für "Maximum Aluminium P". Je nach Herstellungsverfahren kann Zeolith P geringe Anteile anderer Zeolithe enthalten. Sehr reiner Zeolith P ist in WO 94/26662 beschrieben worden.
Im Rahmen der Erfindung lassen sich auch solche feinteiligen, wasserunlöslichen Natriumalumosilikate verwenden, die in Gegenwart von wasserlöslichen anorganischen oder organischen Dispergiermitteln gefällt und kristallisiert wurden. Diese können in beliebiger Weise vor oder während der Fällung bzw. Kristallisation in das Reaktionsgemisch eingebracht werden.
Ganz besonders bevorzugt sind Na-Zeolith A und Na-Zeolith P.
Die Hydrotalcite und/oder Zeolithe können in Mengen von beispielsweise 0,1 bis 20, zweckmäßig 0,1 bis 10 und insbesondere 0,1 bis 5 Gew.-Teilen, bezogen auf
100 Gew.-Teile halogenhaltiges Polymer angewandt werden.

Die erfindungsgemässen Zusammensetzungen können auch mit weiteren üblichen Zusatzstoffen versetzt sein, wie etwa Stabilisierungs-, Hilfs- und Verarbeitungsmitteln, z. B. Alkali- und Erdalkaliverbindungen, Gleitmitteln, Weichmachem, Pigmenten, Füllstoffen, Phosphiten, Thiophosphiten und Thiophosphaten, Mercaptocarbonsäureestern, epoxidierten Fettsäureestern, Antioxidantien, UV-Absorbern und Lichtschutzmitteln, optischen Aufhellern, Schlagzähmodifikatoren und Verarbeitungshilfen, Geliermitteln, Antistatika, Biociden, Metalldesaktivatoren, Flammschutz- und Treibmitteln, Antifogagents, Kompatibilisatoren sowie Antiplateoutagents. (Vgl. "Handbook of PVC-Formulating" von E. J. Wickson, John Wiley & Sons, New York 1993). Es folgen Beispiele für derartige Zusatzstoffe:

**I. Füllstoffe:** Füllstoffe (HANDBOOK OF PVC FORMULATING E. J. Wickson,
John Wiley & Sons, Inc., 1993, SS. 393- 449) und Verstärkungsmittel (TASCHENBUCH der KUNSTSTOFFADDITIVE, R. Gächter & H. Müller, Carl Hanser, 1990, SS. 549 - 615) (wie beispielsweise Calciumcarbonat, Dolomit, Wollastonit, Magnesiumoxid, Magnesiumhydroxid, Silikate, China-Clay, Talk, Glasfasern, Glaskugeln, Holzmehl, Glimmer, Metalloxide, oder Metallhydroxide, Ruß, Graphit, Gesteinsmehl, Schwerspat, Glasfasern, Talk, Kaolin und Kreide verwandt. Bevorzugt ist Kreide. Die Füllstoffe können in einer Menge von vorzugsweise mindestens
1 Teil, beispielsweise 5 bis 200, zweckmäßig 10 bis 150 und insbesondere 15 bis
100 Gew.-Teilen, bezogen auf 100 Gew.-Teile PVC, eingesetzt werden.

**II. Metallseifen:** Metallseifen sind in der Hauptsache Metallcarboxylate bevorzugt längerkettiger Carbonsäuren. Geläufige Beispiele sind Stearate und Laurate, auch Oleate und Salze kürzerkettiger Alkancarbonsäuren. Als Metallseifen sollen auch Alkylbenzoesäuren gelten. Als Metalle seien genannt: Li, Na, K, Mg, Ca, Sr, Ba, Zn, Al, La, Ce und Seltenerdenmetalle. Oft verwendet man sog. synergistische Mischungen wie Barium/Zink-, Magnesium/Zink-, Calcium/Zink- oder Calcium/Magnesium/Zink-Stabilisatoren. Die Metallseifen können einzeln oder in Mischungen eingesetzt werden. Eine Übersicht über gebräuchliche Metallseifen findet sich in Ullmanns Encyclopedia of Industrial Chemistry, 5^{th} Ed., Vol. A16 (1985), S. 361 ff.). Zweckmäßig verwendet man organische Metallseifen aus der Reihe der aliphatischen gesättigten C₂-C₂₂-Carboxylate, der aliphatischen ungesättigten
C₃-C₂₂-Carboxylate, der aliphatischen C₂-C₂₂-Carboxylate, die mit wenigstens einer OH-Gruppe substituiert sind, der cyclischen und bicyclischen Carboxylate mit
5 - 22 C-Atomen, der unsubstituierten, mit wenigstens einer OH-Gruppe substituierten und/oder C₁-C₁₆-alkylsubstituierten Benzolcarboxylate, der unsubstituierten, mit wenigstens einer OH-Gruppe substituierten und/oder C₁-C₁₆-alkylsubstituierten Naphthalincarboxylate, der Phenyl-C₁-C₁₆-alkylcarboxylate, der Naphthyl-C₁-C₁₆-alkylcarboxylate oder der gegebenenfalls mit C₁-C₁₂-Alkyl substituierten Phenolate, Tallate und Resinate. Namentlich zu erwähnen sind, als Beispiele, die Zink-, Calcium-, Magnesiumoder Bariumsalze der monovalenten Carbonsäuren, wie Essigsäure, Propionsäure, Buttersäure, Valeriansäure, Hexansäure, Önanthsäure, Octansäure, Neodecansäure, 2-Ethylhexansäure, Pelargonsäure, Decansäure, Undecansäure, Dodecansäure, Tridecansäure, Myristylsäure, Palmitinsäure, Isostearinsäure, Stearinsäure, 12-Hydroxystearinsäure, Behensäure, Benzoesäure,
p-tert-Butylbenzoesäure, N,N,-Dimethylhydroxybenzoesäure,
3,5-Di-tert-butyl-4-hydroxybenzoesäure, Tolylsäure, Dimethylbenzoesäure, Ethylbenzoesäure, n-Propylbenzoesäure, Salicylsäure,
p-tert-Octylsalicylsäure, und Sorbinsäure; Calcium-, Magnesium- und Zinksalze der Monoester der divalenten Carbonsäuren, wie Oxalsäure, Malonsäure, Bernsteinsäure, Glutarsäure, Adipinsäure, Fumarsäure, Pentan-1,5-dicarbonsäure,
Hexan-1,6-dicarbonsäure, Heptan-1,7-dicarbonsäure, Octan-1,8-dicarbonsäure, Phthalsäure, Isophthalsäure, Terephthalsäure und Hydroxyphthalsäure; und der Di- oder Triester der tri-oder tetravalenten Carbonsäuren, wie Hemimellithsäure, Trimellithsäure, Pyromellithsäure, Zitronensäure.
Bevorzugt sind Calcium-, Magnesium- und Zink-Carboxylate von Carbonsäuren mit 7 bis 18 C-Atomen (Metallseifen im engeren Sinn), wie beispielsweise Benzoate oder Alkanoate, bevorzugt Stearat, Oleat, Laurat, Palmitat, Behenat, Hydroxystearate, Dihydroxystearate oder 2-Ethylhexanoat. Besonders bevorzugt sind Stearat, Oleat und p-tert-Butylbenzoat. Auch überbasische Carboxylate wie überbasisches Zinkoctoat sind bevorzugt. Ebenfalls bevorzugt sind überbasische Calciumseifen.
Gegebenenfalls kann auch ein Gemisch von Carboxylaten unterschiedlicher Struktur eingesetzt werden.

Bevorzugt sind Zusammensetzungen, wie beschrieben, enthaltend eine organische Zink- oder/und Calciumverbindung.
Neben den genannten Verbindungen kommen auch organische Aluminium-Verbindungen in Frage, außerdem Verbindungen analog den oben erwähnten, insbesondere Aluminium-tri-stearat, Aluminium-di-stearat und Aluminium-monostearat, sowie Aluminiumacetat als auch davon abgeleitete basische Derivate. Zu den verwendbaren und bevorzugten Aluminium-Verbindungen finden sich weitere Erläuterungen in US 4,060,512 und US 3,243,394.

Neben den bereits genannten Verbindungen kommen ferner auch organische Seltenerd-Verbindungen, insbesondere Verbindungen analog den oben erwähnten, in Frage. Unter dem Begriff Seltenerd-Verbindung sind vor allem Verbindungen der Elemente Cer, Praseodym, Neodym, Samarium, Europium, Gadolinium, Terbium, Dysprosium, Holmium, Erbium, Thulium, Ytterbium, Lutetium, Lanthan und Yttrium zu verstehen, wobei Gemische insbesondere mit Cer bevorzugt sind. Weitere bevorzugte Seltenerd-Verbindungen finden sich in der EP-A-0 108 023.

Gegebenenfalls kann ein Gemisch von Zink-, Alkali-, Erdalkali-, Aluminium-, Cer-, Lanthan- oder Lanthanoid-Verbindung unterschiedlicher Struktur eingesetzt werden. Auch können organische Zink-, Aluminium-, Cer-, Alkali-, Erdalkali-, Lanthan- oder Lanthanoid-Verbindungen auf eine Alumosalz-Verbindung gecoatet sein; siehe hierzu auch DE-A-4 031 818.

Die Metallseifen bzw. deren Mischungen können in einer Menge von beispielsweise 0,001 bis 10 Gew.-Teilen, zweckmäßig 0,01 bis 8 Gew.-Teilen, besonders bevorzugt 0,05 bis 5 Gew.-Teilen, bezogen auf 100 Gew.-Teile PVC, angewandt werden. Gleiches gilt für die weiteren Metallstabilisatoren:

**III. Weitere Metallstabilisatoren:** Hier sind vor allem die Organozinnstabilisatoren zu nennen. Insbesondere kann es sich um Carboxylate, Mercaptide und Sulfide handeln. Beispiele geeigneter Verbindungen sind in US 4,743,640 beschrieben.

**IV. Alkali und Erdalkali-Verbindungen:** Darunter versteht man vornehmlich die Carboxylate der oben beschriebenen Säuren, aber auch entsprechende Oxide bzw. Hydroxide oder Carbonate. Es kommen auch deren Gemische mit organischen Säuren in Frage. Beispiele sind LiOH, NaOH, KOH, CaO, Ca(OH₂), MgO, Mg(OH)₂, Sr(OH)₂, Al(OH)₃, CaCO₃ und MgCO₃ (auch basische Carbonate, wie beispielsweise Magnesia Alba und Huntit), sowie fettsaure Naund K-Salze. Bei Erdalkali- und Zn-Carboxylaten können auch deren Addukte mit MO oder M(OH)₂
(M = Ca, Mg, Sr oder Zn), sogenannte "overbased" Verbindungen, zum Einsatz kommen. Bevorzugt werden zusätzlich zur erfindungsgemäßen Stabilisatorkombination Alkali-, Erdalkali- und/oder Aluminiumcarboxylate eingesetzt.

**V. Gleitmittel:** Als Gleitmittel kommen beispielsweise in Betracht:
Montanwachs, Fettsäureester, PE-Wachse, Amidwachse, Chlorparaffine, Glycerinester oder Erdalkaliseifen. Verwendbare Gleitmittel sind auch in "Kunststoffadditive",
R. Gächter/H. Müller, Carl Hanser Verlag, 3. Aufl., 1989, Seiten 478 - 488 beschrieben. Zu erwähnen sind ferner Fettketone (wie in DE 4 204 887 beschrieben) sowie Gleitmittel auf Silikonbasis (wie in EP 0 225 261 beschrieben) oder Kombinationen davon, wie in EP 0 259 783 aufgeführt. Bevorzugt ist Calciumstearat. Die Gleitmittel können auch auf eine Alumosalz-Verbindung aufgebracht werden; siehe hierzu auch DE-A-4 031 818.

**VI. Weichmacher** Als organische Weichmacher kommen beispielsweise solche aus den folgenden Gruppen in Betracht:
A) Phthalsäureester: Beispiele für solche Weichmacher sind Dimethyl-, Diethyl-, Dibutyl-, Dihexyl-, Di-2-ethylhexyl-, Di-n-octyl-, Di-iso-octyl-, Di-iso-nonyl-,
   Di-iso-decyl-, Di-iso-tridecyl-, Dicyclohexyl-, Di-methylcyclohexyl-, Dimethylglycol-, Dibutylglycol-, Benzylbutyl- und Diphenyl-phthalat sowie Mischungen von Phthalaten wie C₇-C₉- und C₉-C₁₁-Alkylphthalate aus überwiegend linearen Alkoholen,
   C₆-C₁₀-n-Alkylphthalate und C₈-C₁₀-n-Alkylphthalate. Bevorzugt sind davon Dibutyl-, Dihexyl-, Di-2-ethylhexyl-, Di-n-octyl-, Di-iso-octyl-, Di-iso-nonyl-,
   Di-iso-decyl-, Di-iso-tridecyl- und Benzylbutyl-phthalat sowie die genannten Mischungen von Alkylphthalaten. Besonders bevorzugt sind
   Di-2-ethylhexyl-, Di-iso-nonyl- und Di-iso-decylphthalat, die auch unter den gebräuchlichen Abkürzungen DOP (Dioctylphthalat, Di-2-ethylhexyl-phthalat), DINP (Diisononylphthalat), DIDP (Diisodecylphthalat) bekannt sind.
B) Ester aliphatischer Dicarbonsäuren, insbesondere Ester von Adipin-, Azelain- und Sebazinsäure: Beispiele für solche Weichmacher sind Di-2-ethylhexyladipat,
   Di-isooctyladipat (Gemisch), Di-iso-nonyladipat (Gemisch), Di-iso-decyladipat (Gemisch), Benzylbutyladipat, Benzyloctyladipat, Di-2-ethylhexylazelat, Di-2-ethylhexylsebacat und Di-iso-decylsebacat (Gemisch). Bevorzugt sind Di-2-ethylhexyladipat und Di-iso-octyladipat.
C) Trimellithsäureester, beispielsweise Tri-2-ethylhexyltrimellithat, Tri-isodecyltrimellithat (Gemisch), Tri-iso-tridecyltrimellithat, Tri-iso-octyltrimellithat (Gemisch) sowie Tri-C₆-C₈-alkyl, Tri-C₆-C₁₀-alkyl-, Tri-C₇-C₉-alkyl-, und Tri-C₉-C₁₁-alkyl-trimellithate. Die letztgenannten Trimellithate entstehen durch Veresterung der Trimellithsäure mit den entsprechenden Alkanolgemischen. Bevorzugte Trimellithate sind Tri-2-ethylhexyltrimellithat und die genannten Trimellithate aus Alkanolgemischen. Gebräuchliche Abkürzungen sind TOTM (Trioctyltrimellitat, Tri-2-ethylhexyl―trimellitat), TIDTM (Triisodecyltrimellitat) und TITDTM (Triisotridecyltrimellitat).
D) Epoxyweichmacher: In der Hauptsache sind das epoxidierte ungesättigte Fettsäuren wie z. B. epoxidiertes Sojabohnenöl.
E) Polymerweichmacher: Eine Definition dieser Weichmacher und Beispiele für solche sind in "Kunststoffadditive", R. Gächter/H. Müller, Carl Hanser Verlag, 3. Aufl., 1989, Kapitel 5.9.6, Seiten 412 - 415, sowie in "PVC Technology ", W. V. Titow,
   4^{th}. Ed., Elsevier Publ., 1984, Seiten 165 - 170 angegeben. Die gebräuchlichsten Ausgangsmaterialien für die Herstellung der Polyesterweichmacher sind: Dicarbonsäuren wie Adipin-, Phthal-, Azelain- und Sebacinsäure; Diole wie 1,2-Propandiol, 1,3-Butandiol, 1,4-Butandiol, 1,6-Hexandiol, Neopentylglycol und Diethylenglykol.
F) Phosphorsäureester: Eine Definition dieser Ester ist im vorstehend genannten "Taschenbuch der Kunststoffadditive" Kapitel 5.9.5, SS. 408- 412, zu finden. Beispiele für solche Phosphorsäureester sind Tributylphosphat, Tri-2-ethylbutylphosphat, Tri-2-ethylhexylphosphat, Trichlorethylphosphat, 2-Ethyl-hexyl-di-phenylphosphat, Kresyldiphenylphosphat, Triphenylphosphat, Trikresylphosphat und Trixylenylphosphat. Bevorzugt sind Tri-2-ethylhexylphosphat sowie ®Reofos 50 und 95 (Ciba Spezialitätenchemie).
G) Chlorierte Kohlenwasserstoffe (Paraffine)
H) Kohlenwasserstoffe
I) Monoester, z. B. Butyloleat, Phenoxyethyloleat, Tetrahydrofurfuryloleat und Alkylsulfonsäureester.
J) Glykolester, z. B. Diglykolbenzoate.
   Definitionen und Beispiele für Weichmacher der Gruppen G) bis J) sind den folgenden Handbüchern zu entnehmen:
   "Kunststoffadditive", R. Gächter/H. Müller, Carl Hanser Verlag, 3. Aufl., 1989, Kapitel 5.9.14.2, SS.422 - 425, (Gruppe G), und Kapitel 5.9.14.1, S. 422, (Gruppe H).
   "PVC Technology", W. V. Titow, 4^{th.} Ed., Elsevier Publishers, 1984, Kapitel 6.10.2,
   Seiten 171 - 173, (Gruppe G), Kapitel 6.10.5 Seite 174, (Gruppe H), Kapitel 6.10.3,
   Seite 173, (Gruppe I) und Kapitel 6.10.4, Seiten 173 - 174 (Gruppe J).
   Es können auch Mischungen unterschiedlicher Weichmacher verwandt werden. Die Weichmacher können in einer Menge von beispielsweise 5 bis 20 Gew.-Teilen, zweckmäßig 10 bis 20 Gew.-Teilen, bezogen auf 100 Gew.-Teile PVC, angewandt werden. Hart- bzw. Halbhart-PVC enthält bevorzugt bis zu 10 %, besonders bevorzugt bis zu 5 % oder keinen Weichmacher.

**VII. Pigmente:** Geeignete Stoffe sind dem Fachmann bekannt. Beispiele für anorganische Pigmente sind TiO₂, Pigmente auf Zirkonoxidbasis, BaSO₄, Zinkoxid (Zinkweiss) und Lithopone (Zinksulfid/Bariumsulfat), Ruß, Russ-Titandioxid-Mischungen, Eisenoxidpigmente, Sb₂O₃, (Ti,Ba,Sb)O₂, Cr₂O₃, Spinelle wie Cobaltblau und Cobaltgrün, Cd(S,Se), Ultramarinblau. Organische Pigmente sind
z. B. Azopigmente, Phthalocyaninpigmente, Chinacridonpigmente, Perylenpigmente, Diketo-pyrrolopyrrolpigmente und Anthrachinonpigmente. Bevorzugt ist TiO₂ auch in mikronisierter Form. Eine Definition und weitere Beschreibungen finden sich im "Handbook of PVC Formulating", E. J.Wickson, John Wiley & Sons, New York, 1993.

**VIII. Phosphite (Phosphorigsäuretriester):** Beispiele sind Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert-butylphenyl)-phosphit, Diisodecylpentaerythrit―diphosphit, Bis-(2,4-di-tert-butylphenyl)-pentaerythritdiphosphit, Bis-(2,6-di-tert-butyl-4-methylphenyl)-pentaerythritdiphosphit, Bis-isodecyloxy-pentaerythritdiphosphit, Bis-(2,4-di-tert-butyl-6-methylphenyl)-pentaerythritdiphosphit, Bis-(2,4,6-tri-tert-butylphenyl)-pentaerythritdiphosphit, Tristearyl-sorbit―triphosphit, Bis-(2,4-di-tert-butyl-6-methylphenyl)-methylphosphit, Bis-(2,4-di-tert―butyl-6-methylphenyl)-ethylphosphit. Besonders geeignete sind Trioctyl-, Tridecyl-, Tridodecyl-, Tritetradecyl, Tristearyl-, Trioleyl-, Triphenyl-, Trikresyl-, Tris-p-nonylphenyl- oder Tricyclohexylphosphit und besonders bevorzugt sind die Aryl-Dialkyl- sowie die Alkyl-Diaryl-Phosphite, wie z. B. Phenyldidecyl-, (2,4-Di-tert-butylphenyl)-didodecylphosphit, (2,6-Di-tert- butylphenyl)-di-dodecylphosphit und die Dialkylund Diaryl-pentaerythrit-diphosphite, wie Distearylpentaerythrit-diphosphit, sowie nichtstöchiometrische Triarylphosphite. z. B. der Zusammensetzung (H₁₉C₉-C₆H₄)O_{1,5}P(OC_{12,13}H_{25,27})_{1,5} oder (H₈C₁₇-C₆H₄)O₂P(i-C₈H₁₇O) oder (H₁₉C₉-C₆H₄)O_{1,5}P(OC_{9,11}H_{19,23})_{1,5} oder

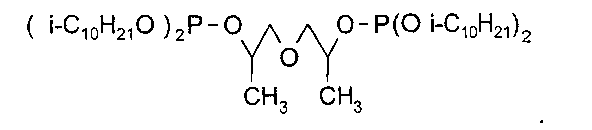

Bevorzugte organische Phosphite sind Distearyl-pentaerythrit-diphosphit, Trisnonylphenylphosphit und Phenyl-didecyl-phosphit. Als weiteres kommen Phosphorigsäurediester (mit o.g. Resten) sowie Phosphorigsäuremonoester (mit o.g. Resten) evtl. auch als Alkali-, Erdalkali-, Zink- oder Aluminiumsalz in Betracht. Diese Phosphorigsäureester können auch auf eine Alumosalz-Verbindung aufgebracht sein; siehe hierzu auch DE-A-4 031 818.
Die organischen Phosphite können in einer Menge von beispielsweise 0,01 bis 10, zweckmäßig 0,05 bis 5 und insbesondere 0,1 bis 3 Gew.-Teilen, bezogen auf
100 Gew.-Teile PVC, angewandt werden.

**IX. Thiophosphite und Thiophosphate:** Unter Thiophosphiten bzw.Thiophosphaten sind Verbindungen vom allgemeinen Typ: (RS)₃P, (RS)₃P=O bzw. (RS)₃P=S zu verstehen, wie sie etwa in den Patentschriften DE 2 809 492,
EP 0 090 770 und EP 0 573 394 beschrieben werden. Beispiele für diese Verbindungen sind: Trithiohexylphosphit, Trithiooctylphosphit, Trithio-laurylphosphit, Trithiobenzylphosphit, Trithiophosphorigesäure-tris-(carbo-i-octyloxy)-methylester. Trithiophosphorigsäure-tris-(carbo-trimethylcyclohexyloxy)-methylester, Trithio-phosphorsäure-S,S,S-tris-(carbo-i-octyloxy)-methylester, Trithiophosphorsäure-S,S,S-tris-(carbo-2-ethylhexyloxy)-methylester, Trithiophosphorsäure-S,S,S-tris-1-(carbo-hexyloxy)-ethylester, Trithiophosphorsäure-S,S,S-tris-1-(carbo-2-ethylhexyloxy)-ethalester, Trithiophosphorsäure-S,S,S-tris-2-(carbo-2-ethylhexyloxy)-ethylester.

**X. Mercaptocarbonsäure-Ester:** Beispiele für diese Verbindungen sind: Ester der Thioglycolsäure, Thioäpfelsäure, Mercaptopropionsäure, der Mercaptobenzoesäuren bzw. der Thiomilchsäure, Mercaptoethylstearat und - oleat, wie sie in den Patenten FR 2 459 816, EP0 090 748, FR 2 552 440, EP 0 365 483 beschrieben sind. Die gen. Mercaptocarbonsäure-Ester umfassen auch Polyolester bzw. deren Partialester, sowie davon abgeleitete Thioether.

**XI. Epoxidierte Fettsäureester und andere Epoxidverbindungen:** Die erfindungsgemäße Stablisatorkombination kann zusätzlich vorzugsweise mindestens einen epoxidierten Fettsäureester enthalten. Es kommen dafür vor allem Ester von Fettsäuren aus natürlichen Quellen (Fettsäureglyceride), wie Sojaöl oder Rapsöl, in Frage. Es können aber auch synthetische Produkte zum Einsatz kommen, wie epoxidiertes Butyloleat. Ebenso verwendet werden können epoxidiertes Polybutadien und Polyisopren, gegebenenfalls auch in partiell hydroxylierter Form, oder Glycidylacrylat und Glycidylmethacrylat als Homo- bzw. Copolymer. Diese Epoxyverbindungen können auch auf eine Alumosalz-Verbindung aufgebracht sein; siehe hierzu auch DE-A-4 031 818.

**XII. Antioxidantien:** Als solche kommen beispielsweise in Betracht:
Alkylierte Monophenole, z. B. 2,6-Di-tert-butyl-4-methylphenol, 2-tert-butyl-4,6-dimethylphenol, 2,6-Di-tert-butyl-4-ethylphenol, 2,6-Di-tert-butyl-4-nbutylphenol, 2,6-Di-tert―butyl-4-iso-butylphenol, 2,6-Di-cyclopentyl-4-methylphenol, 2-(alpha-Methylcyclohexyl)-4,6-dimethylphenol, 2,6-Di-octadecyl-4-methylphenol, 2,4,6-Tri-cyclohexylphenol, 2,6-Di―tert-butyl-4-methoxymethylphenol, 2,6-Di-nonyl-4-methylphenol, 2,4-Dimethyl-6-(1'-methylundec-1'-yl)-phenol, 2,4-Dimethyl-6-(1'-methyl-heptadec-1'-yl)-phenol, 2,4-Dimethyl-6- (1'-methyl-tridec-1'-yl)-phenol, Octylphenol, Nonylphenol,Dodecylphenol und Mischungen davon.
Alkylthiomethylphenole, z. B. 2,4-Di-octylthiomethyl-6-tert-butylphenol, 2,4-Di―octylthiomethyl-6-methylphenol, 2,4-Di-octylthiomethyl-6-ethylphenol, 2,6-Didodecylthiomethyl-4-nonylphenol.
Alkylierte Hydrochinone, z. B. 2,6-Di-tert-butyl-4-methoxyphenol, 2,5-Ditert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis-(3,5-di-tert-butyl-4-hydroxyphenyl)adipat.
Hydroxylierte Thiodiphenylether, z. B. 2,2'-Thio-bis-(6-tert-butyl-4-methylphenol), 2,2'―Thio-bis-(4-octylphenol), 4,4'-Thio-bis-(6-tert-butyl-3-methylphenol), 4,4'―Thio-bis-(6-tert-butyl-2-methylphenol), 4,4'-Thio-bis-(3,6-di-sec.-amylphenol), 4,4'-Bis-(2,6-dimethyl-4-hydroxyphenyl)-disulfid.
Alkyliden-Bisphenole, z. B. 2,2'-Methylen-bis-(6-tert-butyl-4-methylphenol), 2,2'―Methylen―bis-(6-tert-butyl-4-ethylphenol), 2,2'-Methylen-bis-[4-methyl-6-(alpha―methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert-butylphenol), 2,2'-Ethyliden-bis-(4,6-di-tert-butylphenol), 2,2'-Ethyliden-bis-(6-tert-butyl-4-isobutylphenol), 2,2'-Methylen-bis-[6-(alpha-methylbenzyl)-4-nonylphenol], 2,2'-Methylen-bis-[6-(alpha,alpha-dimethylbenzyl)-4-nonylphenol], 4,4'-Methylen-bis-(2,6-di-tert-butylphenol), 4,4'-Methylen-bis-(6-tert-butyl-2-methylphenol), 1,1-Bis-(5-tert-butyl-4-hydroxy-2-methylphenyl)-butan, 2,6-Bis-(3-tert-butyl-5-methyl-2-hydroxybenzyl)-4-methylphenol, 1,1,3-Tris-(5-tert-butyl-4-hydroxy-2-methylphenyl)-butan, 1,1-Bis-(5-tert-butyl-4-hydroxy-2-methyl-phenyl)-3-n-dodecylmercaptobutan, Ethylenglycol-bis-[3,3-bis-(3'-tert-butyl-4'-hydroxyphenyl)-butyrat], Bis-(3-tert-butyl-4-hydroxy-5-methyl-phenyl)-dicyclopentadien, Bis-[2-(3'-tert-butyl-2'―hydroxy-5'-methyl-benzyl)-6-tert-butyl-4-methyl-phenyl]-terephthalat, 1,1-Bis-(3,5-dimethyl-2-hydroxyphenyl)-butan, 2,2-Bis-(3,5-di-tertbutyl-4-hydroxyphenyl)-propan, 2,2-Bis-(4-hydroxyphenyl)-propan, 2,2-Bis-(5-tert-butyl-4-hydroxy-2-methylphenyl)-4-n-dodecylmercapto-butan, 1,1,5,5-Tetra-(5-tert-butyl-4-hydroxy-2-methylphenyl)-pentan.
Benzylverbindungen, z. B. 3,5,3',5'-Tetra-tert-butyl-4,4'-dihydroxydibenzylether, Octadecyl-4-hydroxy-3,5-dimethylbenzyl-mercaptoacetat, Tris-(3,5-di-tert-butyl-4-hydroxybenzyl)-amin, Bis-(4-tert-butyl-3-hydroxy-2,6-dimethylbenzyl)-dithioterephthalat, Bis-(3,5-di-tert-butyl-4-hydroxybenzyl)-sulfid, Isooctyl-3,5-ditert―butyl-4-hydroxybenzyl-mercaptoacetat.
Hydroxybenzylierte Malonate, z. B. Dioctadecyl-2,2-bis-(3,5-di-tert-butyl-2-hydroxybenzyl)-malonat, Di-octadecyl-2-(3-tert-butyl-4-hydroxy-5-methylbenzyl)-malonat, Di―dodecylmercaptoethyl-2,2-bis-(3,5-di-tert-butyl-4-hydroxybenzyl)-malonat, Di-[4-(1,1,3,3-tetramethylbutyl)-phenyl]-2,2-bis-(3,5-di-tert-butyl-4-hydroxybenzyl)-malonat.
Hydroxybenzyl-Aromaten, z. B. 1,3,5-Tris-(3,5-di-tert-butyl-4-hydroxybenzyl)-2,4,6-trimethylbenzol, 1,4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.
Triazinverbindungen, z. B. 2,4-Bis-octylmercapto-6-(3,5-di-tert-butyl-4-hydroxyanilino)-1,3,5-triazin, 2-Octylmercapto-4,6-bis-(3,5-di-tert-butyl-4-hydroxyanilino)-1,3,5-triazin, 2-Octylmercapto-4,6-bis-(3,5-di-tert-butyl-4-hydroxyphenoxy)- 1,3,5-triazin, 2,4,6-Tris-(3,5-di-tert-butyl-4-hydroxyphenoxy)-1,2,3-triazin, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 2,4,6-Tris-(3,5-ditert-butyl-4-hydroxyphenylethyl)-1,3,5-triazin, 1,3,5-Tris-(3,5-di-tert-butyl-4-hydroxyphenylpropionyl)-hexahydro-1,3,5-triazin, 1,3,5-Tris-(3,5-dicyclohexyl-4-hydroxybenzyl)-isocyanurat.
Phosphonate und Phosphonite, z. B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethyleste rs, Tetrakis-(2,4-di-tert-butylphenyl)-4,4'-biphenylen―diphosphonit, 6-lsooctyloxy-2,4,8,10-tetra-tert-butyl-12H-dibenz[d,g]-1,3,2-dioxaphosphocin, 6-Fluor-2,4,8,10-tetra-tert-butyl-12-methyl-dibenz[d,g]-1,3,2-dioxaphosphocin.
Acylaminophenole, z. B. 4-Hydroxy-laurinsäureanilid, 4-Hydroxystearinsäureanilid, N-(3,5-di-tert-butyl-4-hydroxyphenyl)-carbaminsäureoctylester.
Ester der beta-(3,5-Di-tert-butyl-4-hydroxyphenyl)-propionsäure mit einoder mehrwertigen Alkoholen, wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Dipentaerythrit, Tris-(hydroxyethyl)-isocyanurat, N,N'―Bis-(hydroxyethyl)-oxalsäurediamid, 3-Thiaundecanol, 3-Thiapentadecanol, Trimethylhexandiol, Trimethylolpropan, Di-trimethylolpropan, 4-Hydroxymethyl-1-phospha-2,6,7-trioxabicyclo-[2.2.2]-octan.
Ester der beta-(5-tert-Butyl-4-hydroxy-3-methylphenyl)-propionsäure mit ein- oder mehrwertigen Alkoholen, wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thia-undecanol, 3-Thiapentadecanol, Trimethylhexandiol, Trimethylolpropan, 4-Hydroxymethyl-1-phospha-2,6,7-trioxabicyclo-[2.2.2]-octan.
Ester der beta-(3,5-Dicyclohexyl-4-hydroxyphenyl)-propionsäure mit einoder mehrwertigen Alkoholen, wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thia-undecanol, 3-Thiapentadecanol, Trimethylhexandiol, Trimethylolpropan, 4-Hydroxymethyl-1-phospha-2,6,7-trioxabicyclo-[2.2.2]-octan.
Ester der 3,5-Di-tert-butyl-4-hydroxyphenylessigsäure mit ein- oder mehrwertigen Alkoholen, wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris--(hydroxy)ethyl-isocyanurat, N,N'-Bis-(hydroxyethyl)-oxalsäurediamid, 3-Thia-undecanol, 3-Thiapentadecanol, Trimethylhexandiol, Trimethylolpropan, 4-Hydroxymethyl-1-phospha-2,6,7-trioxabicyclo-[2.2.2]-octan.
Amide der beta-(3,5-Di-tert-butyl-4-hydroxyphenyl)-propionsäure, wie z. B. N,N'-Bis-(3,5-di-tert-butyl-4-hydroxyphenylpropionyl)-hexamethylendiamin, N,N'-Bis-(3,5-di-tert-butyl-4-hydroxyphenylpropionyl)-trimethylendiamin, N,N'-Bis-(3,5-di-tert-butyl-4-hydroxyphenylpropionyl)-hydrazin.
Vitamin E (Tocopherol) und Abkömmlinge
Bevorzugt sind Antioxidantien der Gruppen 1-5, 10 und 12 insbesondere 2,2-Bis-(4-hydroxyphenyl)-propan, Ester der 3,5-Di-tert-butyl-4-hydroxyphenylpropionsäure mit Octanol, Octadecanol oder Pentaerythrit oder Tris-(2,4-di-tert-butylphenyl)-phosphit.
Gegebenenfalls kann auch ein Gemisch von Antioxidantien unterschiedlicher Struktur eingesetzt werden.
Die Antioxidantien können in einer Menge von beispielsweise 0,01 bis 10 Gew.-Teilen, zweckmäßig 0,1 bis 10 Gew.-Teilen und insbesondere 0,1 bis 5 Gew.-Teilen, bezogen auf 100 Gew.-Teile PVC, angewandt werden.

**XIII. UV-Absorber und Lichtschutzmittel:** Beispiele dafür sind:
2-(2'-Hydroxyphenyl)-benztriazole, wie z. B. 2-(2'-Hydroxy-5'-methylphenyl)-benztriazol, 2-(3',5'-Di-tert-butyl-2'-hydroxyphenyl)-benztriazol, 2-(5'-tert-Butyl-2'-hydroxyphenyl)-benztriazol, 2-(2'-Hydroxy-5'-(1,1,3,3-tetramethylbutyl)phenyl)-benztriazol, 2-(3',5'-Di-tert-butyl-2'-hydroxyphenyl)-5-chlor-benztriazol, 2-(3'-tert-Butyl- 2'-hydroxy-5'-methylphenyl)-5-chlor-benztriazol, 2-(3'-sec-Butyl-5'-tert-butyl-2'-hydroxyphenyl)-benztriazol, 2-(2'-Hydroxy-4'-octoxyphenyl)-benztriazol, 2-(3',5'-Di-tert-amyl-2'-hydroxyphenyl)-benztriazol, 2-(3',5'-Bis-(alpha,alpha―dimethylbenzyl)-2'-hydroxyphenyl)-benztriazol, Mischung aus 2-(3'-tert-Butyl-2'-hydroxy-5'-(2-octyloxycarbonylethyl)-phenyl)-5-chlor-benztriazol, 2-(3'-tert-Butyl-5'-[2-(2-ethylhexyloxy)-carbonylethyl]-2'-hydroxyphenyl)-5-chlor-benztriazol, 2-(3'-tert-Butyl-2'-hydroxy-5'-(2-methoxycarbonylethyl)phenyl)-5-chlor-benztriazol, 2-(3'-tert-Butyl-2'-hydroxy-5'-(2-methoxycarbonylethyl)phenyl)-benztriazol, 2-(3'-tert-Butyl-2'-hydroxy-5'-(2-octyloxycarbonylethyl)phenyl)-benztriazol, 2-(3'-tert-Butyl-5'-[2-(2-ethylhexyloxy)carbonylethyl]-2'-hydroxyphenyl)-benztriazol, 2-(3'-Dodecyl-2'-hydroxy-5'―methylphenyl)-benztriazol, und 2-(3'-tert-Butyl-2'-hydroxy-5'-(2-isooctyloxycarbonylethyl)phenyl-benztriazol, 2,2'-Methylen-bis[4-(1,1,3,3-tetramethylbutyl)-6-benztriazol-2-yl-phenol]; Umesterungsprodukt von 2-[3'-tert―Butyl-5'-(2-methoxycarbonylethyl)-2'―hydroxy―phenyl]-benztriazol mit Polyethylenglycol 300; 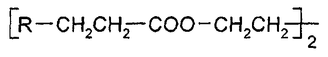 mit R = 3'-tert-Butyl-4'-hydroxy-5'-2H-benztriazol-2-yl-phenyl.
2-Hydroxybenzophenone, wie z. B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.
Ester von gegebenenfalls substituierten Benzoesäuren, wie z. B. 4-tert-Butyl―phenylsalicylat, Phenylsalicylat, Octylphenyl-salicylat, Dibenzoylresorcin, Bis-(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäureoctadecylester, 3,5-Di-tert-butyl-4-hydroxybenzoesäure-2-methyl-4,6-di-tertbutylphenylester.
Acrylate, wie z. B. alpha-Cyan-beta,beta-diphenylacrylsäure-ethylester bzw. -isooctylester, alpha-Carbomethoxy-zimtsäuremethylester, alpha-Cyanobeta-methyl-p-methoxy-zimtsäuremethylester bzw. -butylester, alpha-Carbomethoxy-p-methoxy-zimtsäure-methylester, N-(beta-Carbomethoxy-b-cyanovinyl)-2-methyl-indolin.
Nickelverbindungen, wie z. B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyldiethanolamin, Nickeldibutyldithiocarbamat, Nickelsalze von 4-Hydroxy-3,5-ditert-butylbenzylphosphonsäure―monoalkylestern, wie vom Methyl- oder Ethylester, Nickelkomplexe von Ketoximen, wie von 2-Hydroxy-4-methyl-phenylundecylketoxim, Nickelkomplexe des 1-Phenyl-4-lauroyl-5-hydroxy-pyrazols, gegebenenfalls mit zusätzlichen Liganden.
Oxalsäurediamide, wie z. B. 4,4'-Di-octyloxy-oxanilid, 2,2'-Di-octyloxy-5,5'di-tert―butyl―oxanilid, 2,2'-Di-dodecyloxy-5,5'di-tert-butyl-oxanilid, 2-Ethoxy-2'ethyl―oxanilid, N,N'-Bis-(3-dimethylaminopropyl)-oxalamid, 2-Ethoxy-5-tertbutyl-2'-ethyloxanilid und dessen Gemisch mit 2-Ethoxy-2'-ethyl-5,4'-di-tert-butyloxanilid, Gemische von o- und p-Methoxy- sowie von o- und p-Ethoxy-disubstituierten Oxaniliden.
2-(2-Hydroxyphenyl)-1,3,5-triazine, wie z. B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethylphenyl)-1,3,5-triazin, 2-[2-hydroxy-4-(2-hydroxy-3-octyloxy-propyloxy)phenyl]-4,6-bis(2,4-dimethylphenyl)-1,3,5-triazin.
Sterisch gehinderte Amine, wie z. B. Bis(2,2,6,6-tetramethyl-piperidin-4-yl)-sebacat, Bis(2,2,6,6-tetramethyl-piperidin-4-yl)-succinat, Bis(1,2,2,6,6-pentamethylpiperidin-4-yl)-sebacat, Bis(1-octyloxy-2,2,6,6-tetramethylpiperidin-4-yl)-sebacat, n-Butyl-3,5-di-tert-butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, lineare oder cyclische Kondensationsprodukte aus N,N'-Bis(2,2,6,6-Tetramethyl-4-piperidyl)-hexamethylendiamin und 4-tert-Octylamino-2,6-dichlor-1,3,5-s-triazin, Tris(2,2,6,6-tetramethyl-4-piperidyl)-nitrilotriacetat, Tetrakis(2,2,6,6-tetramethyl-4-piperidyl)-1,2,3,4-butantetraoat, 1,1'-(1,2-Ethandiyl)-bis(3,3,5,5-tetramethylpiperazinon), 4-Benzoyl-2,2,6,6-tetramethylpiperidin, 4-Stearyloxy-2,2,6,6-tetramethylpiperidin, Bis(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis(1-octyloxy-2,2,6,6-tetramethylpiperidyl)-sebacat, Bis(1-octyloxy-2,2,6,6-tetramethylpiperidyl)-succinat, lineare oder cyclische Kondensationsprodukte aus N,N'-Bis(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis(3-aminopropylamino)ethan, Kondensationsprodukt aus 2-Chlor-4,6-di-(4-n-butylamino-1,2,2,6,6-pentamethylpiperidyl)-1,3,5-triazin und 1,2-Bis(3-aminopropylamino)-ethan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion, Gemisch von 4-Hexadecyloxy- und 4-Stearyloxy-2,2,6,6-tetramethylpiperidin, Kondensationsprodukt aus N,N'-Bis(2,2,6,6-tetramethyl-4-piperidyl)-hexamethylendiamin und 4-Cyclohexylamino-2,6-dichlor-1,3,5-triazin, Kondensationsprodukt aus 1,2-Bis(3-aminopropylamino)-ethan und 2,4,6-trichlor-1,3,5-triazin sowie 4-Butylamino-2,2,6,6-tetramethyl-piperidin (CAS Reg. No. [136504-96-6]); N-(2,2,6,6-tetramethyl-4-piperidyl)-n-dodecylsuccinimid, N-(1,2,2,6,6-pentamethyl-4-piperidyl)-n-dodecylsuccinimid, 2-Undecyl-7,7,9,9-tetramethyl-1-oxa-3,8-diaza-4-oxo-spiro[4,5]decan, Umsetzungsprodukt von 7,7,9,9-Tetramethyl-2-cycloundecyl-1-oxa-3,8-diaza-4-oxospiro[4,5]decan und Epichlorhydrin, 1,1-Bis(1,2,2,6,6-pentamethyl-4-piperidyloxycarbonyl)-2-(4-methoxyphenyl)-ethen, N,N'-Bis-formyl-N,N'-bis(2,2,6,6-tetramethyl-4-piperidyl)-hexamethylendiamin, Diester der 4-Methoxy-methylen-malonsäure mit 1,2,2,6,6-Pentamethyl-4-hydroxy-piperidin, Poly[methylpropyl-3-oxy-4-(2,2,6,6-tetramethyl-4-piperidyl)]-siloxan, Reaktionsprodukt aus Maleinsäureanhydrid-aolefin-copolymer und 2,2,6,6-Tetramethyl-4-aminopiperidin oder 1,2,2,6,6-Pentamethyl-4-aminopiperidin.

**XIV. Treibmittel:** Treibmittel sind z. B. organische Azo- und Hydrazoverbindungen, Tetrazole, Oxazine, Isatosäureanhydrid, sowie Soda und Natriumbicarbonat. Bevorzugt sind Azodicarbonamid und Natriumbicarbonat sowie deren Mischungen.
Definitionen und Beispiele für Schlagzähmodifikatoren und Verarbeitungshilfen, Geliermittel, Antistatika, Biocide, Metalldesaktivatoren, optische Aufheller, Flammschutzmittel, Antifogging-agents sowie Kompatibilisatoren sind beschrieben in "Kunststoffadditive", R. Gächter/H. Müller, Carl Hanser Verlag, 3. Aufl., 1989, und im "Handbook of Polyvinyl Chloride Formulating" E. J. Wickson, J. Wiley & Sons, 1993, sowie in "Plastics Additives" G. Pritchard, Chapman & Hall, London, 1st Ed., 1998.
Schlagzähmodifikatoren sind ferner ausführlich beschrieben in "Impact Modifiers for PVC", J. T. Lutz/D. L. Dunkelberger, John Wiley & Sons, 1992.

**XV. beta-Diketone, beta-Ketoester:** Verwendbare 1,3-Dicarbonylverbindungen können lineare oder cyclische Dicarbonylverbindungen sein. Bevorzugt werden Dicarbonylverbindungen der folgenden Formel verwandt: R'₁COCHR₂'-COR'₃
worin R'₁ C₁-C₂₂-Alkyl, C₅-C₁₀-Hydroxyalkyl, C₂-C₁₈-Alkenyl, Phenyl, durch OH, C₁-C₄-Alkyl, C₁-C₄-Alkoxy oder Halogen substituiertes Phenyl, C₇-C₁₀-Phenylalkyl, C₅-C₁₂-Cycloalkyl, durch C₁-C₄-Alkyl substituiertes C₅-C₁₂-Cycloalkyl oder eine Gruppe -R'₅-S-R'₆ oder -R'₅-O-R'₆ bedeutet, R'₂ Wasserstoff, C₁-C₈-Alkyl,
C₂-C₁₂-Alkenyl, Phenyl, C₇-C₁₂-Alkylphenyl, C₇-C₁₀-Phenylalkyl oder eine Gruppe -CO-R'₄ bedeutet, R'₃ eine der für R'₁ gegebenen Bedeutungen hat oder
C₁-C₁₈-Alkoxy bedeutet, R'₄ C₁-C₄-Alkyl oder Phenyl bedeutet,
R'₅ C₁-C₁₀-Alkylen bedeutet und R'₆ C₁-C₁₂-Alkyl, Phenyl, C₇-C₁₈-Alkylphenyl oder C₇-C₁₀-Phenylalkyl bedeutet.
Hierzu gehören die Hydroxylgruppen enthaltenden Diketone der EP 0 346 279 und die Oxa- und Thia-diketone der EP 0 307 358 ebenso wie die auf Isocyansäure basierenden Ketoester der US 4,339,383.
R'₁ und R'₃ als Alkyl können insbesondere C₁-C₁₈-Alkyl sein, wie z. B. Methyl, Ethyl, n-Propyl, Isopropyl, n-Butyl, tert Butyl, Pentyl, Hexyl, Heptyl, Octyl, Decyl, Dodecyl oder Octadecyl.
R'₁ und R'₃ als Hydroxyalkyl stellen insbesondere eine Gruppe -(CH₂)ₙ-OH dar, worin n 5, 6 oder 7 ist.
R'₁ und R'₃ als Alkenyl können beispielsweise Vinyl, Allyl, Methallyl, 1-Butenyl, 1-Hexenyl oder Oleyl bedeuten, vorzugsweise Allyl.
R'₁ und R'₃ als durch OH, Alkyl, Alkoxy oder Halogen substituiertes Phenyl können beispielsweise Tolyl, Xylyl, tert Butylphenyl, Methoxyphenyl, Ethoxyphenyl, Hydroxyphenyl, Chlorphenyl oder Dichlorphenyl sein.
R'₁ und R'₃ als Phenylalkyl sind insbesondere Benzyl. R'₂ und R'₃ als Cycloalkyl oder Alkyl―cycloalkyl sind insbesondere Cyclohexyl oder Methylcyclohexyl.
R'₂ als Alkyl kann insbesondere C₁-C₄-Alkyl sein. R'₂ als C₂-C₁₂-Alkenyl kann insbesondere Allyl sein. R'₂ als Alkylphenyl kann insbesondere Tolyl sein. R'₂ als Phenylalkyl kann insbesondere Benzyl sein. Vorzugsweise ist R'₂ Wasserstoff. R'₃ als Alkoxy kann z. B. Methoxy, Ethoxy, Butoxy, Hexyloxy, Octyloxy, Dodecyloxy, Tridecyloxy, Tetradecyloxy oder Octadecyloxy sein. R'₅ als C₁-C₁₀-Alkylen ist insbesondere C₂-C₄-Alkylen. R'₆ als Alkyl ist insbesondere C₄-C₁₂-Alkyl, wie z. B. Butyl, Hexyl, Octyl, Decyl oder Dodecyl. R'₆ als Alkylphenyl ist insbesondere Tolyl. R'₆ als Phenylalkyl ist insbesondere Benzyl.
Beispiele für 1,3-Dicarbonylverbindungen der obigen Formel sowie deren Alkali-, Erdalkali- und Zinkchelate sind Acetylaceton, Butanoylaceton, Heptanoylaceton, Stearoylaceton, Palmitoylaceton, Lauroylaceton, 7-tert.-Nonylthio-heptandion-2,4, Benzoylaceton, Dibenzoylmethan, Lauroylbenzoylmethan, Palmitoylbenzoylmethan, Stearoyl-benzoylmethan, Isooctylbenzoylmethan, 5-Hydroxycapronyl-benzoylmethan, Tribenzoylmethan, Bis(4-methylbenzoyl)methan, Benzoyl-p-chlorbenzoylmethan, Bis(2-hydroxybenzoyl)methan, 4-Methoxybenzoyl-benzoylmethan, Bis(4-methoxybenzoyl)-methan, 1-Benzoyl-1-acetylnonan, Benzoyl-acetyl-phenylmethan, Stearoyl-4-methoxybenzoylmethan, Bis(4-tert-butylbenzoyl)methan, Benzoyl-formylmethan, Benzoyl-phenylacetylmethan, Bis-cyclohexanoyl-methan, Di-pivaloyl-methan, 2-Acetylcyclopentanon, 2-Benzoylcyclo-pentanon, Diacetessigsäuremethyl-, -ethylund -allylester, Benzoyl-, Propionyl- und Butyryl-acetessigsäuremethyl- und -ethylester, Triacetylmethan, Acetessigsäuremethyl-, -ethyl-, -hexyl-, -octyl-, -dodecyl- oder -octadecylester, Benzoylessigsäuremethyl-, -ethyl-, -butyl-, -2-ethylhexyl-, -dodecyl- oder -octadecylester, sowie Propionyl- und Butyrylessigsäure-C₁-C₁₈-alkylester. Stearoylessigsäureethyl-, -propyl-, -butyl-, -hexyl- oder -octylester sowie mehrkernige β-Ketoester wie in EP 0 433 230 beschrieben und Dehydracetsäure sowie deren Zink-, Magnesium- oder Alkalisalze.
Bevorzugt sind 1,3-Diketoverbindungen der obigen Formel, worin R'₁ C₁-C₁₈-Alkyl, Phenyl, durch OH, Methyl oder Methoxy substituiertes Phenyl, C₇-C₁₀-Phenylalkyl oder Cyclohexyl ist, R'₂ Wasserstoff ist und R'₃ eine der für R'₁ gegebenen Bedeutungen hat.
Die 1,3-Diketoverbindungen können in einer Menge von beispielsweise 0,01 bis 10, zweckmäßig 0,01 bis 3 und insbesondere 0,01 bis 2 Gew.-Teilen, bezogen auf
100 Gew.-Teile PVC, angewandt werden.

Beispiele für die zu stabilisierenden chlorhaltige Polymere sind: Polymere des Vinylchlorides, Vinylidenchlorids, Vinylharze, enthaltend Vinylchlorideinheiten in deren Struktur, wie Copolymere des Vinylchlorids und Vinylester von aliphatischen Säuren, insbesondere Vinylacetat, Copolymere des Vinylchlorids mit Estern der Acryl- und Methycrylsäure und mit Acrylnitril, Copolymere des Vinylchlorids mit Dienverbindungen und ungesättigten Dicarbonsäuren oder deren Anhydride, wie Copolymere des Vinylchlorids mit Diethylmaleat, Diethylfumarat oder Maleinsäureanhydrid, nachchlorierte Polymere und Copolymere des Vinylchlorids, Copolymere des Vinylchlorids und Vinylidenchlorids mit ungesättigten Aldehyden, Ketonen und anderen, wie Acrolein, Crotonaldehyd, Vinylmethylketon, Vinylmethylether, Vinylisobutylether und ähnliche; Polymere des Vinylidenchlorids und Copolymere desselben mit Vinylchlorid und anderen polymerisierbaren Verbindungen; Polymere des Vinylchloracetates und Dichlordivinylethers; chlorierte Polymere des Vinylacetates, chlorierte polymerische Ester der Acrylsäure und der alphasubstituierten Acrylsäure; Polymere von chlorierten Styrolen, zum Beispiel Dichlorstyrol; Chlorkautschuke; chlorierte Polymere des Ethylens; Polymere und nachchlorierte Polymere von Chlorbutadiens und deren Copolymere mit Vinylchlorid, chlorierte Natur- und Synthesekautschuke, sowie Mischungen der genannten Polymere unter sich oder mit anderen polymerisierbaren Verbindungen. Im Rahmen dieser Erfindung sind unter PVC auch Copolymerisate mit polymerisierbaren Verbindungen wie Acrylnitril, Vinylacetat oder ABS zu verstehen, wobei es sich um Suspensions-, Masse- oder Emulsionspolymerisate handeln kann. Bevorzugt ist ein PVC-Homopolymer, auch in Kombination mit Polyacrylaten.
Ferner kommen auch Pfropfpolymerisate von PVC mit EVA, ABS und MBS in Betracht. Bevorzugte Substrate sind auch Mischungen der vorstehend genannten Homo-und Copolymerisate, insbesondere Vinylchlorid-Homopolymerisate, mit anderen thermoplastischen oder/und elastomeren Polymeren, insbesondere Blends mit ABS, MBS, NBR, SAN, EVA, CPE, MBAS, PMA, PMMA, EPDM und Polylactonen.
Beispiele für solche Komponenten sind Zusammensetzungen aus (i) 20-80 Gew.-Teilen eines Vinylchlorid-Homopolymeren (PVC) und (ii) 80-20 Gew.-Teile mindestens eines thermoplastischen Copolymerisats auf der Basis von Styrol und Acrylnitril, insbesondere aus der Gruppe ABS, NBR, NAR, SAN und EVA. Die verwandten Abkürzungen für die Copolymerisate sind dem Fachmann geläufig und bedeuten folgendes: ABS: Acrylnitril-Butadien-Styrol; SAN: Styrol-Acrylnitril; NBR: Acrylnitril-Butadien; NAR: Acrylnitril-Acrylat; EVA: EthylenVinylacetat. Es kommen insbesondere auch Styrol-Acrylnitril-Copolymerisate auf Acrylat-Basis (ASA) in Betracht. Bevorzugt als Komponente sind in diesem Zusammenhang Polymerzusammensetzungen, die als Komponenten (i) und (ii) eine Mischung aus
25 - 75 Gew.-% PVC und 75 - 25 Gew.-% der genannten Copolymerisate enthalten. Beispiele für solche Zusammensetzungen sind: 25 - 50 Gew.-% PVC und
75 - 50 Gew.-% Copolymerisate bzw. 40 - 75 Gew.-% PVC und 60 - 25 Gew.-% Copolymerisate. Bevorzugte Copolymerisate sind ABS, SAN und modifiziertes EVA, insbesondere ABS. Besonders geeignet sind auch NBR, NAR und EVA. In der erfindungsgemäßen Zusammensetzung können eines oder mehrere der genannten Copolymerisate vorhanden sein. Von besonderer Bedeutung sind als Komponente Zusammensetzungen, die (i) 100 Gewichtsteile PVC, und (ii) 0 - 300 Gewichtsteile ABS und/oder mit SAN modifiziertes ABS und 0 - 80 Gewichtsteile der Copolymeren NBR, NAR und/oder EVA, insbesondere jedoch EVA, enthalten.
Weiterhin kommen zur Stabilisierung im Rahmen dieser Erfindung auch insbesondere Recyclate chlorhaltiger Polymere in Frage, wobei es sich hierbei um die oben näher beschriebenen Polymere handelt, welche durch Verarbeitung, Gebrauch oder Lagerung eine Schädigung erfahren haben.

Besonders bevorzugt ist PVC-Recyclat. In den Recyclaten können auch kleine Mengen an Fremdstoffen enthalten sein, wie z. B. Papier, Pigmente, Klebstoffe, die oft schwierig zu entfernen sind. Diese Fremdstoffe können auch aus dem Kontakt mit diversen Stoffen während des Gebrauchs oder der Aufarbeitung stammen, wie z. B. Treibstoffreste, Lackanteile, Metallspuren und Initiatorreste.

Die erfindungsgemäße Stabilisierung ist besonders bei PVC-Formulierungen von Vorteil, wie sie für Rohre und Profile üblich sind. Die Stabilisierung kann ohne Schwermetallverbindungen (Sn-, Pb-, Cd-, Zn-Stabilisatoren) durchgeführt werden. Diese Eigenschaft bietet auf bestimmten Gebieten Vorteile, weil Schwermetalle - mit Ausnahmen von allenfalls Zink - sowohl bei der Produktion als auch bei der Anwendung bestimmter PVC-Artikel aus ökologischen Gründen oft unerwünscht sind. Auch bereitet die Herstellung von Schwermetallstabilisatoren aus gewerbehygienischer Hinsicht oftmals Probleme. Ebenfalls ist die Verhüttung von schwermetallhaltigen Erzen sehr oft mit gravierenden Einflüssen auf die Umwelt verbunden, wobei Umwelt das Biosystem Mensch, Tier (Fisch), Pflanze, Luft und Boden mit einschließt. Aus diesen Gründen ist auch die Verbrennung bzw. Deponierung von schwermetallhaltigen Kunststoffen umstritten.

Die Erfindung betrifft auch ein Verfahren zur Stabilisierung von PVC, dadurch gekennzeichnet, daß man diesem mindestens eine der oben erwähnten Stabilisatorkombinationen zufügt.

Zweckmäßig kann die Einarbeitung der Stabilisatoren nach folgenden Methoden erfolgen: als Emulsion oder Dispersion (Eine Möglichkeit ist z. B. die Form einer pastösen Mischung. Ein Vorteil der erfindungsgemäßen Kombination besteht bei dieser Darreichungsform in der Stabilität der Paste.); als Trockenmischung während des Vermischens von Zusatzkomponenten oder Polymermischungen; durch direktes Zugeben in die Verarbeitungsapparatur (z. B. Kalander, Mischer, Kneter, Extruder und dergleichen) oder als Lösung oder Schmelze bzw. als Flakes oder Pellets in staubfreier Form als One- Pack.
Das erfindungsgemäß stabilisierte PVC, das die Erfindung ebenfalls betrifft, kann auf an sich bekannte Weise hergestellt werden, wozu man unter Verwendung an sich bekannter Vorrichtungen wie der oben genannten Verarbeitungsapparaturen die erfindungsgemäße Stabilisatorkombination und gegebenenfalls weitere Zusätze mit dem PVC vermischt. Hierbei können die Stabilisatoren einzeln oder in Mischung zugegeben werden oder auch in Form sogenannter Masterbatches.
Das nach vorliegender Erfindung stabilisierte PVC kann auf bekannte Weisen in die gewünschte Form gebracht werden. Solche Verfahren sind beispielsweise Mahlen, Kalandrieren, Extrudieren, Spritzgießen oder Spinnen, ferner Extrusions-Blasen. Das stabilisierte PVC kann auch zu Schaumstoffen verarbeitet werden.
Ein erfindungsgemäß stabilisiertes PVC eignet sich z. B. besonders für Hohlkörper (Flaschen), Verpackungsfolien (Tiefziehfolien), Blasfolien, Rohre, Schaumstoffe, Schwerprofile (Fensterrahmen), Lichtwandprofile, Bauprofile, Sidings, Fittings, Bürofolien und Apparatur-Gehäuse (Computer, Haushaltsgeräte).
Bevorzugt sind PVC-Hartschaumstoff-Formkörper und PVC-Rohre wie für Trinkoder Abwasser, Druckrohre, Gasrohre, Kabelkanal- und Kabelschutzrohre, Rohre für Industrieleitungen, Sickerrohre, Abflußrohre, Dachrinnenrohre und Drainagerohre. Näheres hierzu siehe "Kunststoffhandbuch PVC", Band 2/2, W. Becker/H. Braun,
2. Aufl., 1985, Carl Hanser Verlag, Seiten 1236 - 1277.

6-Aminouracile werden nach bekannten Methoden hergestellt [z. B. US Patent 2,598,936, WO 96/04280, J. Org. Chem. **16,** 1879- 1890 (1951), J. Org. Chem. **30,** 656 (1965), JACS **82,** 3973 (1960) und
Synthesis 1996, Seite 459 ff., Berichte **99** 3530 (1966) und CA 70 87727j (1969)].
Dargestellte Verbindungen 1 bis 22 sind in der **Tabelle 1** zusammengefasst.

Teile und Prozentangaben beziehen sich wie im übrigen Text auf das Gewicht, sofern nicht anders angegeben.

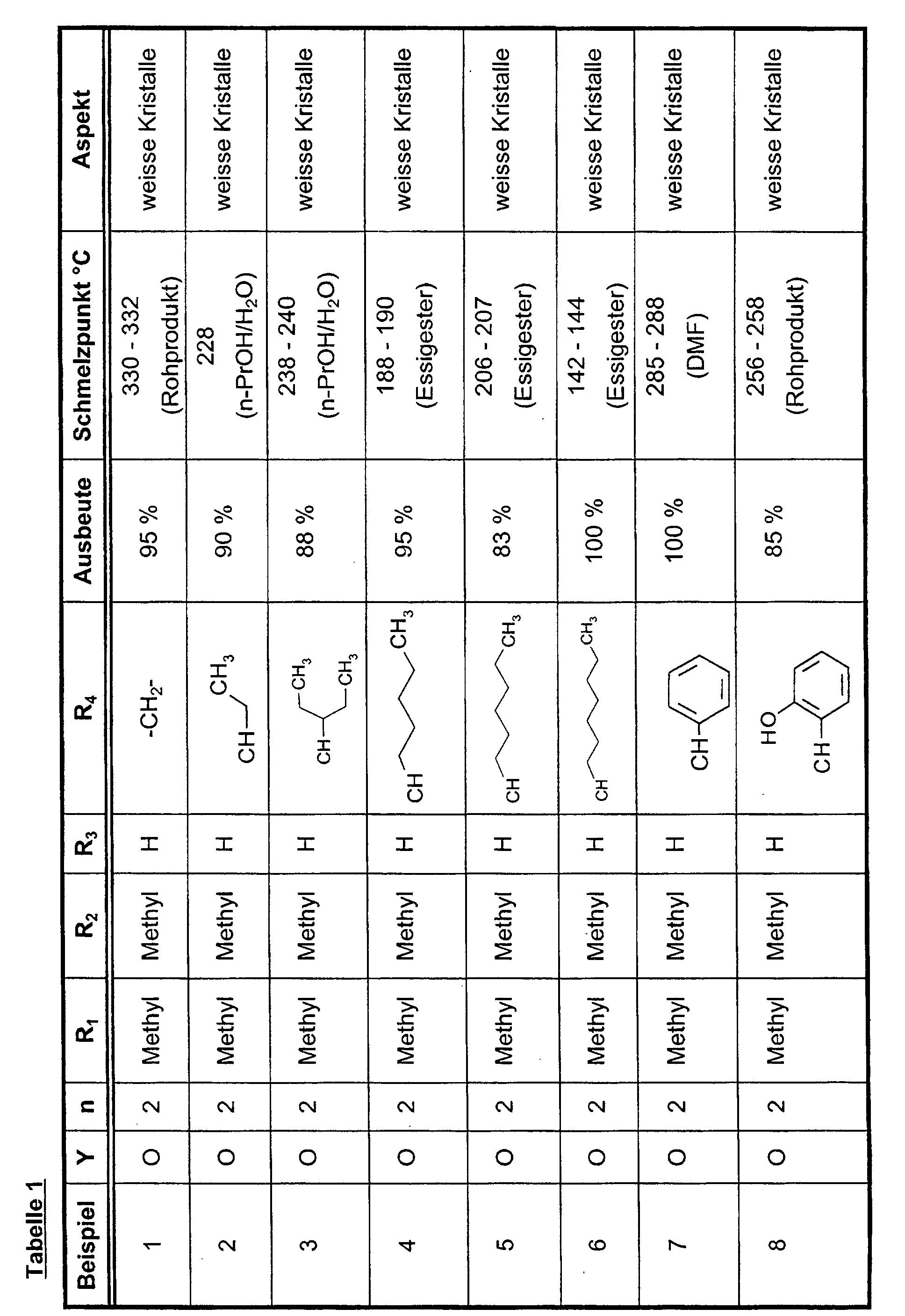

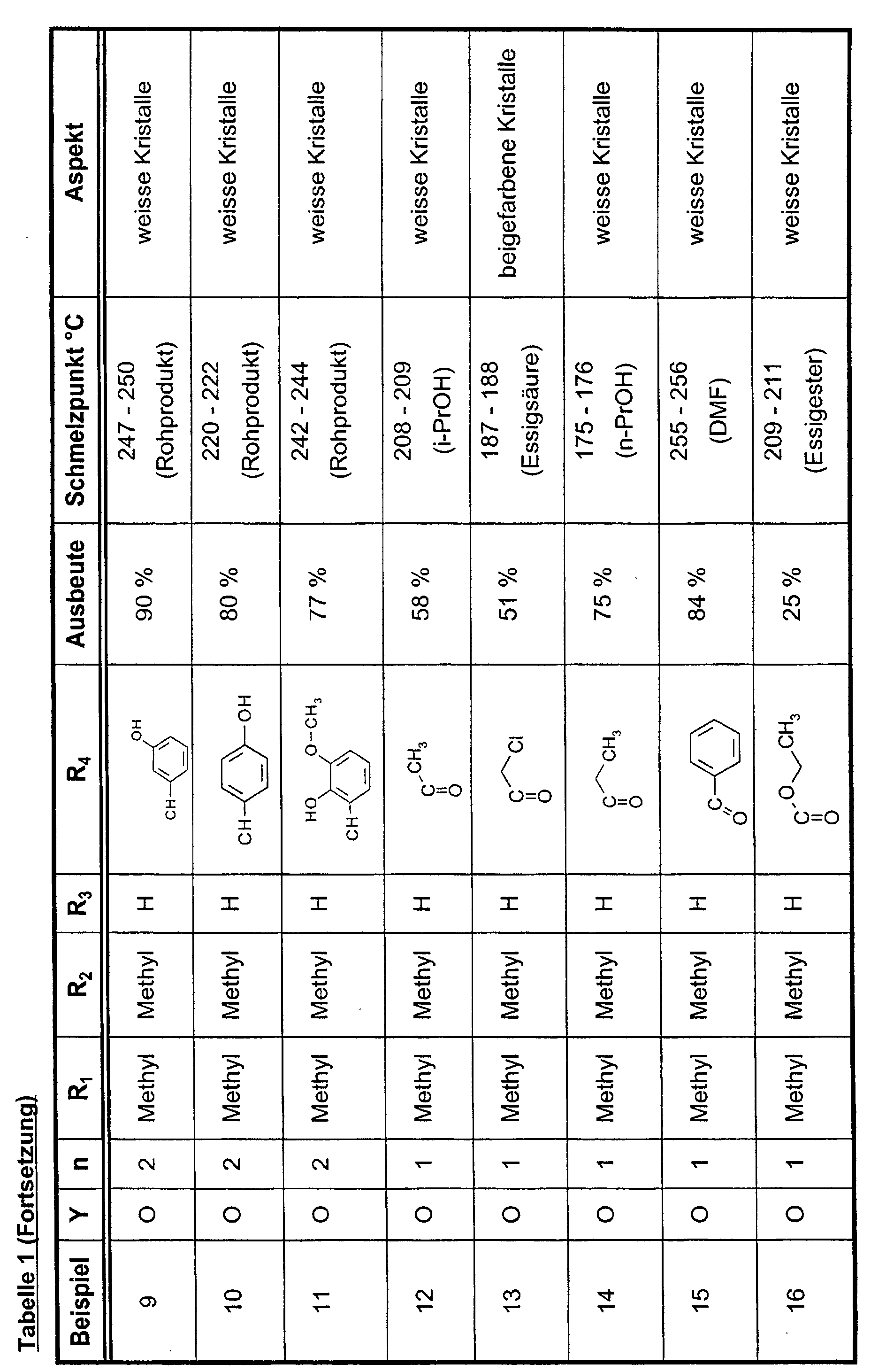

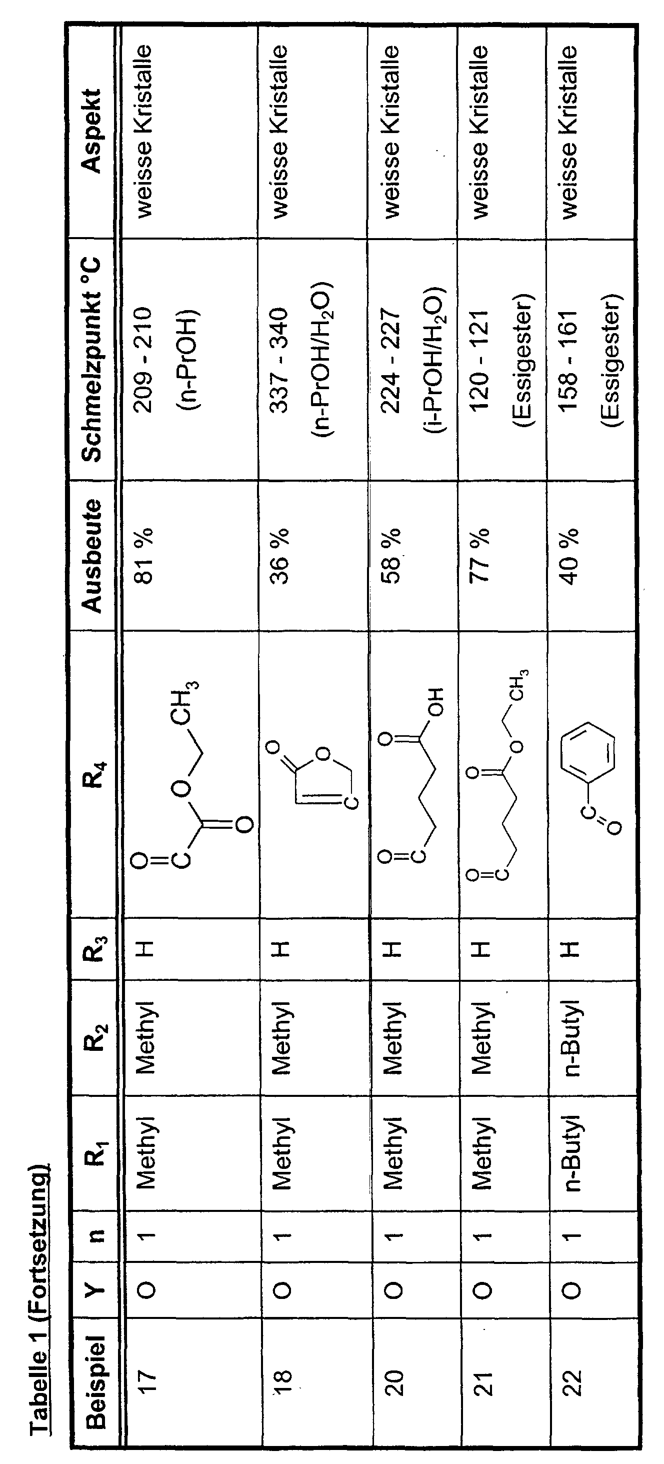

### Herstellungsbeispiele

### Beispiel 1

Ein Gemisch aus 31,0 g (0,2 Mol) 6-Amino-1,3-dimethyluracil, 8,1 g (0,11 Mol) 37 %-iger Formaldehydlösung, 120 ml Toluol und 100 ml Eisessig werden solange am Rückfluss erwärmt, bis sich 15 ml eines Wasser/Essigsäure-Gemisches in einem Wasserabscheider gesammelt haben. Anschliessend wird auf 20 °C abgekühlt, der erhaltene Niederschlag abgesaugt, mit Toluol gewaschen und im Vakuum bei 100 °C getrocknet.

Ausbeute: 30,6 g = 95 % d. Th.

### Beispiel 15

Es werden 15,5 g (0,1 Mol) 6-Amino-1,3-dimethyluracil, 14,0 g (0,1 Mol) Benzoylchlorid und 16 g Pyridin 4 Stunden unter Rühren auf 116 °C erwärmt. Nach dem Abkühlen auf 20 °C wird das Reaktionsgemisch in 250 ml Wasser eingerührt, der ausgefallene Niederschlag nach 2 Stunden abgesaugt, mit Wasser gewaschen und im Vakuum-Trockenschrank bei 100 °C getrocknet.

Ausbeute: 21,7 g = 84 % d. Th.

### Beispiel 18

In einem 500 ml 2-Halskolben mit Magnetrührer, Thermometer, Wasserabscheider und Rückflusskühler werden 34 g (0,22 Mol) 6-Amino-1,3-dimethyluracil, 45 g (0,3 Mol) 4-Chloracetessigsäuremethylester, 80 ml Essigsäure und 100 ml Toluol 3,5 Stunden unter Rühren zum Rückfluss erwärmt. Hierbei werden 10,2 ml Wasser/Essigsäure-Gemisch abgenommen. Anschliessend wird das Reaktionsgemisch auf 30 °C (Niederschlagsbildung) und folgend auf 0 °C abgekühlt. Der Niederschlag wird isoliert, mit Wasser gewaschen und getrocknet.

Ausbeute: 18,7 g = 35,8 % d. Th.

### Beispiel 18

Es werden 15,5 g (0,1 Mol) 6-Amino-1,3-dimethyluracil, 26,4 g (0,1 Mol) 4-(Dimethylaminoethylen)-2,6-di-tert.-butylphenol und 100 ml Essigsäure 2,5 Stunden unter Rühren auf 121 °C erwärmt. Nach dem Abkühlen auf 20 °C wird das Reaktionsgemisch in 500 ml Wasser eingerührt, der ausgefallene Niederschlag abgesaugt, mit Wasser gewaschen und im Vakuum-Trockenschrank bei 100 °C getrocknet.

Ausbeute: 37,3 g = 100 % d. Th.

### Beispiel 20

31,0 g (0,2 Mol) 6-Amino-1,3-dimethyluracil, 25,0 g (0,22 Mol) Glutarsäureanhydrid und 100 ml Essigsäure werden 90 Minuten auf 120 °C erwärmt und anschließend auf 20°C abgekühlt. Das Reaktionsgemisch wird in 500 ml Wasser eingerührt, der weiße Niederschlag abgesaugt, mit Wasser gewaschen und bis zur Gewichtskonstanz getrocknet.

Ausbeute: 31,2 g = 57,9 % d. Th.

### Beispiel 21

18,8 g (0,07 Mol) der Verbindung aus Beispiel 20 werden 3 Stunden mit 100 ml Ethanol und 2 ml konzentrierter Schwefelsäure am Rückfluß gerührt, dann auf 20°C abgekühlt und die Reaktionslösung anschliessend in 500 ml Wasser eingerührt.
Es wird mit Natriumhydrogencarbonat neutralisiert und das ausgefallene Reaktionsprodukt abgesaugt, mit Wasser gewaschen und getrocknet.

Ausbeute: 16,0 g = 76,9 % d. Th.

### Beispiel 22

Eine Schmelze von 15,4 g (0,06 Mol) 6-Amino-1,3-di-n-butyluracil und 29,4 g (0,13 Mol) Benzoesäureanhydrid wird 4 Stunden bei 110-120°C gerührt, anschliessend abgekühlt und in 20 ml Methanol gelöst. Diese Lösung wird in eine Lösung von 250 ml Wasser/8 g Natriumhydroxid eingerührt. Der rotbraune Feststoff wird abgesaugt, getrocknet und aus 30 ml Essigsäureethylester umkristallisiert.

Ausbeute: 8,3 g =40,3% d. Th.

### Statischer Hitzetest

Eine Trockenmischung bestehend aus

| | |
|---|---|
| 100,0 Teile | Evipol SH 6030 = PVC K-Wert 60 |
| 5,0 Teile | ESO = epoxidiertes Sojabohnenöl |
| 0,4 Teile | Loxiol G 71 S = hochmolekularer Mehrkomponentenester |
| 0,8 Teile | Irgastab CH 300 = flüssiges Diaryldialkylphosphit |

und jeweils einer der in den Tabellen 2 und 3 (entsprechend den Beispielen aus Tabelle 1) angegebenen Stabilisatoren wird auf einem Mischwalzwerk 5 Minuten bei 180°C gewalzt. Vom gebildeten Walzfell werden Testfolienstücke von 0,3 mm Dicke entnommen. Die Folienproben werden in einem Ofen bei 190°C thermisch belastet. Im zeitlichen Abstand von 5 Minuten wird der Yellowness Index (**YI**) nach ASTM D-1925-70 bestimmt. Die Ergebnisse sind den folgenden Tabellen 2 [1,0 Gewichsteile Stabilisator] und 3 [0,6 Gewichsteile Stabilisator] zu entnehmen. Geringe **YI**-Werte bedeuten gute Stabilisierung.

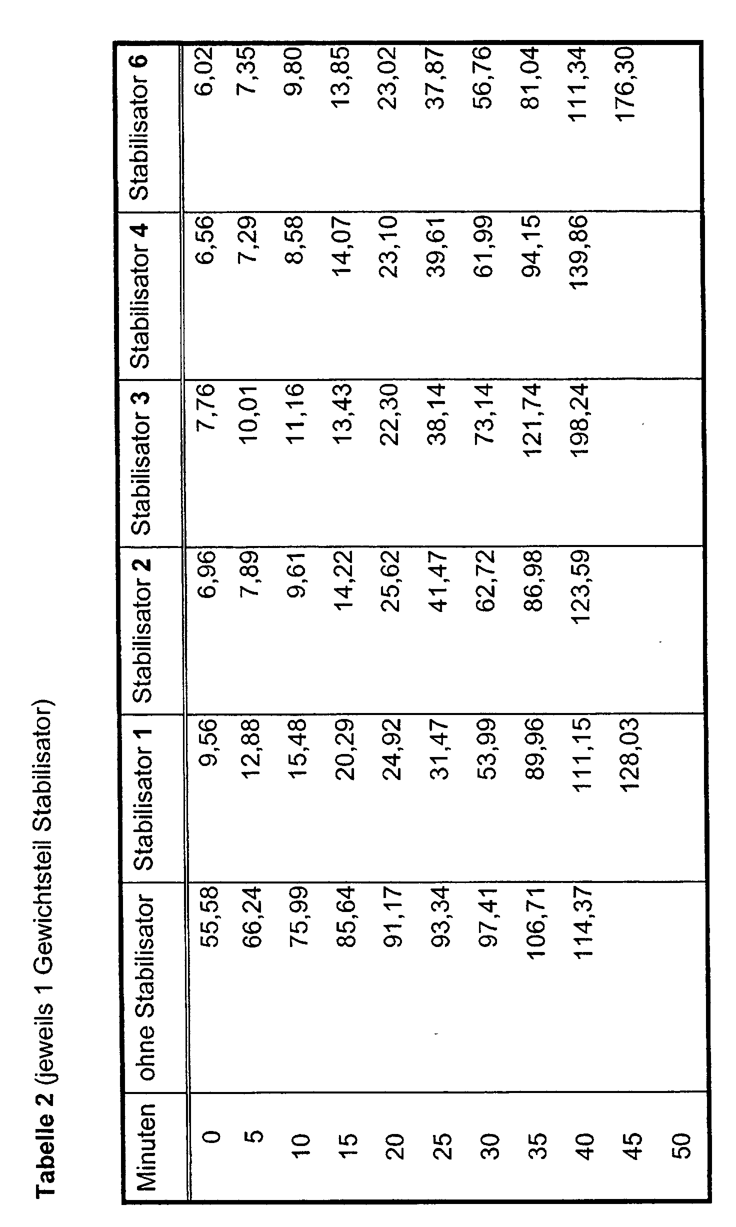

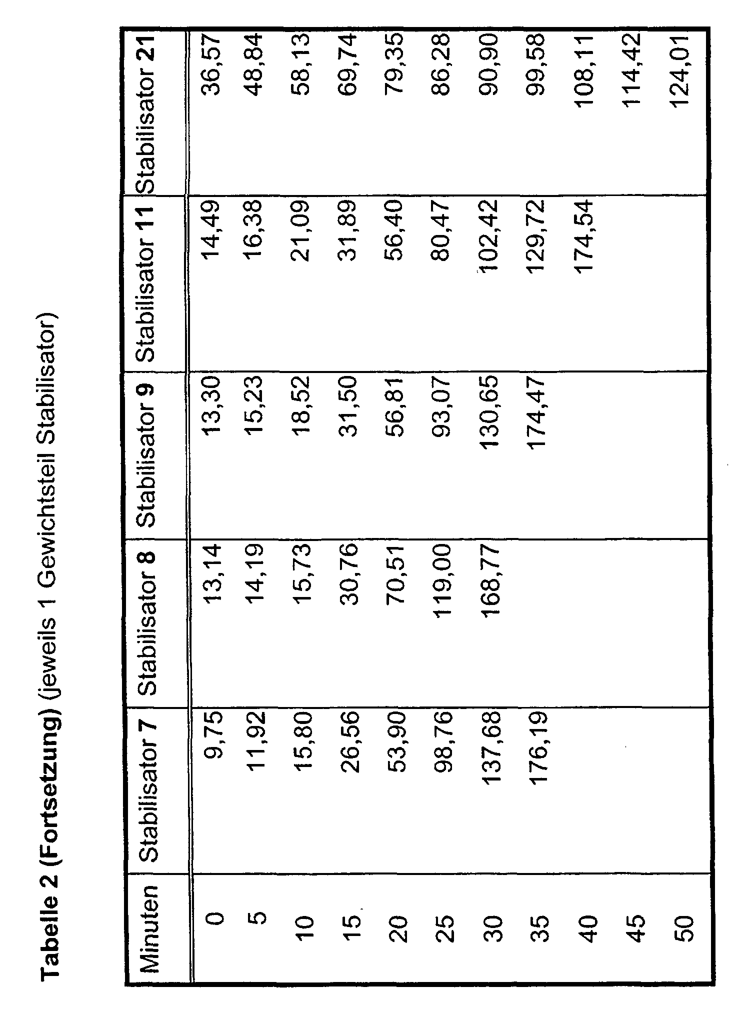

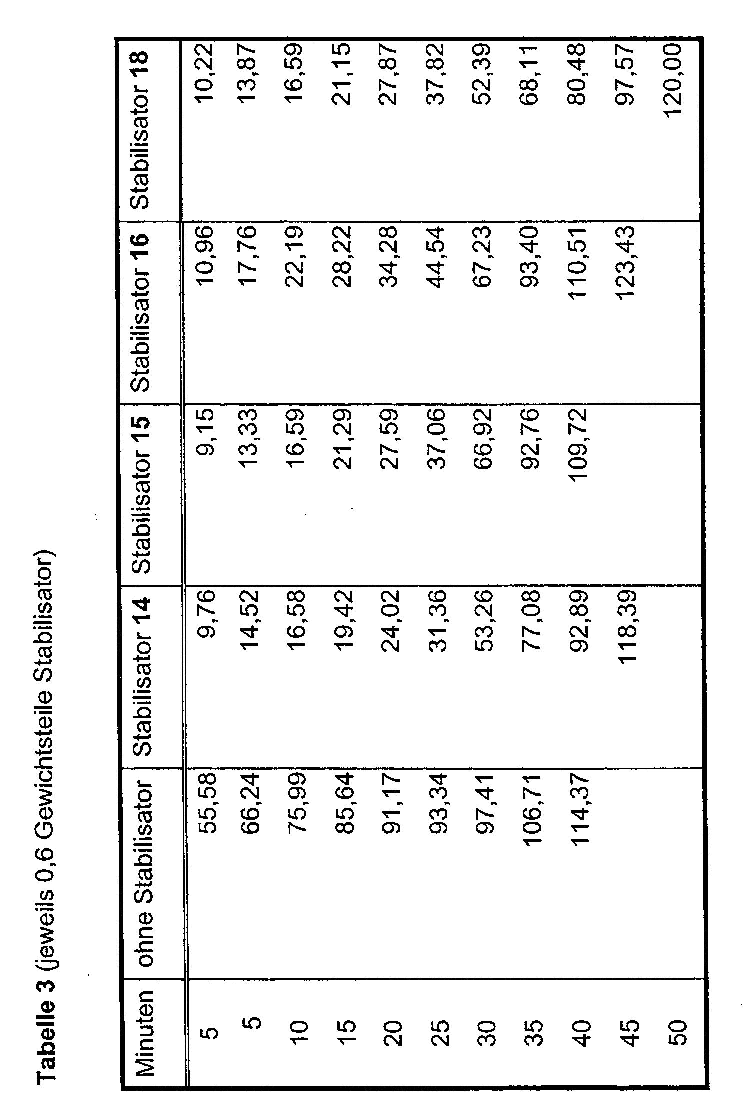

Die Beispiele zeigen deutlich, dass die erfindungsgemässen Stabilisatoren in der Anfangsfarbe, Farbhaltung (Mittelfarbe) und Langzeitstabilität - Messwerte **YI** - verbesserte Ergebnisse gegenüber dem Stand der Technik liefern.

## Patentansprüche

1. Verbindungen der allgemeinen Formel I 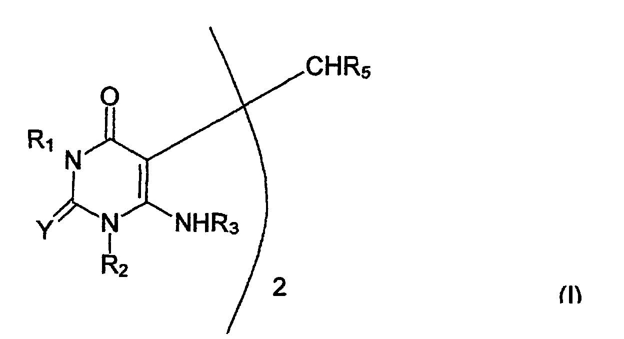 wobei
Y Sauerstoff oder Schwefel ist,
R₁ und R₂ unabhängig voneinander unsubstituiertes oder mit C₁-C₄-Alkoxy-, C₅-C₈-Cycloalkyl-, -OH und/oder Cl substituiertes C₁-C₁₈-Alkyl, C₃-C₆-Alkenyl, C₅-C₈-Cycloalkyl, unsubstituiertes oder am Phenylring mit C₁-C₄-Alkyl-, C₁-C₄-Alkoxy-, C₅-C₈-Cycloalkyl-, -OH und/oder Cl substituiertes C₇-C₉-Phenylalkyl oder Phenyl darstellen,
R₃ H, unsubstituiertes oder mit -OH substituiertes C₁-C₁₈-Alkyl oder unsubstituiertes oder mit -OH substituiertes Phenyl ist, und
R₅ H, C₁-C₁₂-Alkyl, unsubstituiertes oder mit -OH, C₁-C₄-Alkyl, C₁-C₄-Alkoxy-, -C=O(OR₆) und/oder -O-COR₆ substituiertes Phenyl bedeutet und
R₆ verzweigtes oder unverzweigtes C₁-C₁₂-Alkyl oder verzweigtes oder unverzweigtes C₂-C₁₂-Alkenyl ist.

2. Verbindungen der allgemeinen Formel I gemäss Anspruch **1,** wobei Y = Sauerstoff ist.

3. Verbindungen der allgemeinen Formel I gemäss Anspruch **1,** wobei
Y = Sauerstoff und
R₁ und R₂ unabhängig voneinander C₁-C₈-Alkyl, Allyl oder C₇-C₉-Phenylalkyl
darstellen, und

4. Zusammensetzung enthaltend ein chlorhaltiges Polymer und mindestens eine Verbindung der allgemeinen Formel II 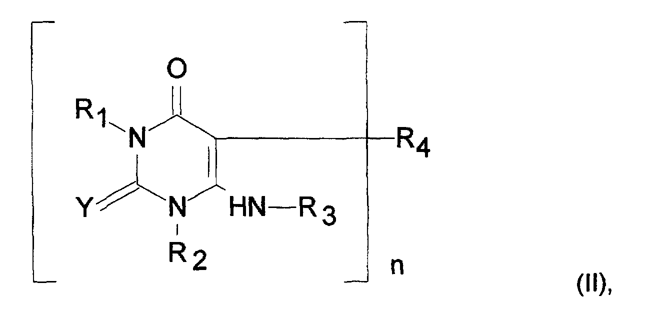 wobei
n 1 oder 2 ist,
Y Sauerstoff oder Schwefel ist,
R₁ und R₂ unabhängig voneinander unsubstituiertes oder mit C₁-C₄-Alkoxy-, C₅-C₈-Cycloalkyl-, -OH und/oder Cl substituiertes C₁-C₁₈-Alkyl, C₃-C₆-Alkenyl, C₅-C₈-Cycloalkyl, unsubstituiertes oder am Phenylring mit C₁-C₄-Alkyl-, C₁-C₄-Alkoxy-, C₅-C₈-Cycloalkyl-, -OH und/oder Cl substituiertes C₇-C₉-Phenylalkyl oder Phenyl darstellen,
R₃ H, unsubstituiertes oder mit -OH substituiertes C₁-C₁₈-Alkyl oder unsubstituiertes oder mit -OH substituiertes Phenyl darstellt, wenn n=1 ist,
R₄ -(C=O)-C₁-C₁₂-Alkyl, -(C=O)-O-C₁-C₁₂-Alkyl, -(C=O)-O-Cₒ-C₁₂-Alkylen-(C=O)OZ,
wobei Z= H oder C₁-C₆-Alkyl ist, -(C=O)-Phenyl, oder 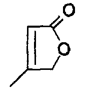 bedeutet,
und, wenn
n 2 ist,
R₄ die Gruppe -CHR₅- bedeutet, wobei
R₅ H, C₁-C₁₂-Alkyl, unsubstituiertes oder mit -OH, C₁-C₄-Alkyl, C₁-C₄-Alkoxy-, -C=O(OR₆) und/oder -O-COR₆ substituiertes Phenyl bedeutet und
R₆ verzweigtes oder unverzweigtes C₁-C₁₂-Alkyl oder verzweigtes oder unverzweigtes C₂-C₁₂-Alkenyl ist.

5. Zusammensetzung gemäss Anspruch 4 enthaltend mindestens einen epoxidierten Fettsäureester.

6. Zusammensetzung gemäss Anspruch 4 enthaltend mindestens ein Zink- und/oder Alkali- und/oder Erdalkalicarboxylat und/oder Aluminiumcarboxylat.

7. Zusammensetzung gemäss Anspruch 4 enthaltend mindestens einen weiteren Stoff aus den Gruppen der Phosphite, Antioxidantien, beta-Dicarbonylverbindungen, Weichmacher, Füllstoffe, Gleitmittel oder Pigmente.

8. Zusammensetzung gemäss Anspruch 4 enthaltend Kreide als Füllstoff.

9. Zusammensetzung gemäss Anspruch 4 enthaltend Calciumstearat als Gleitmittel.

10. Zusammensetzung gemäss Anspruch 4 enthaltend Titandioxid und/oder Zirkonoxid und/oder Bariumsulfat als Pigment.

11. Zusammensetzung gemäss Anspruch 4 enthaltend mindestens ein Polyol und/oder einen Disaccharidalkohol.

12. Zusammensetzung gemäss Anspruch 4 enthaltend mindestens eine Glycidylverbindung.

13. Zusammensetzung gemäss Anspruch 4 enthaltend mindestens eine Perchloratverbindung.

14. Zusammensetzung gemäss Anspruch 4 enthaltend mindestens eine zeolithische Verbindung.

15. Zusammensetzung gemäss Anspruch 4 enthaltend mindestens eine Schichtgitterverbindung.

16. Zusammensetzung gemäss Anspruch 4 enthaltend mindestens ein Hydrotalcit.

17. Verfahren zur Stabilisierung chlorhaltiger Polymere, **dadurch gekennzeichnet, dass** man diesen mindestens eine Verbindung der Formel II gemäss Anspruch 4 einverleibt.

18. Verwendung der Verbindungen der allgemeinen Formel Il gemäss Anspruch 4 als Stabilisatoren von halogenhaltigen Polymeren und deren Recyclaten.

## Claims

1. Compounds of the general formula I 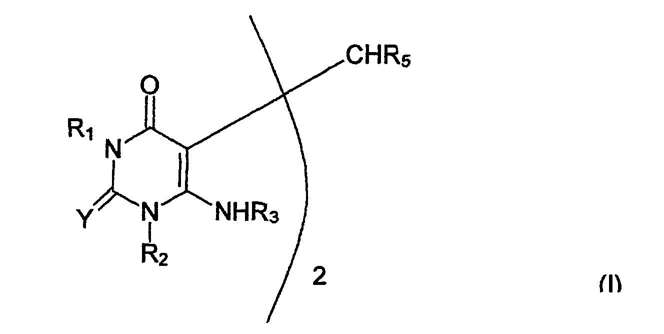 where
Y is oxygen or sulfur,
R₁ and R₂ independently of one another are unsubstituted or C₁-C₄-alkoxy-, C₅-C₈-cycloalkyl-, -OH- and/or Cl-substituted C₁-C₁₈-alkyl, C₃-C₆-alkenyl, C₅-C₈-cycloalkyl, phenyl or C₇-C₉-phenylalkyl which is unsubstituted or substituted on the phenyl ring by C₁-C₄-alkyl-, C₁-C₄-alkoxy-, C₅-C₈-cycloalkyl-, -OH and/or Cl,
R₃ is H, unsubstituted or -OH-substituted C₁-C₁₈-alkyl or unsubstituted or -OH-substituted phenyl, and
R₅ is H, C₁-C₁₂-alkyl, unsubstituted or -OH-, C₁-C₄-alkyl-, C₁-C₄-alkoxy-, -C=O(OR₆)- and/or -O-COR₆-substituted phenyl and
R₆ is branched or unbranched C₁-C₁₂-alkyl or branched or unbranched C₂-C₁₂-alkenyl.

2. Compounds of the general formula I according to Claim 1, where Y is oxygen.

3. Compounds of the general formula I according to Claim 1, where Y is oxygen and
R₁ and R₂ independently of one another are C₁-C₈-alkyl, allyl or C₇-C₉-phenylalkyl.

4. Composition comprising a chlorine-containing polymer and at least one compound of the general formula II 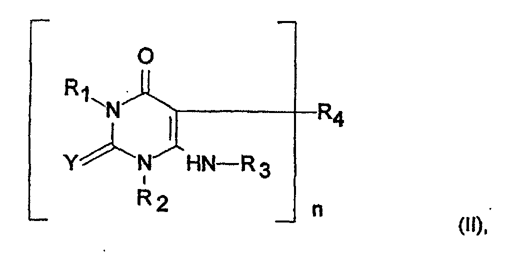 where
n is 1 or 2,
Y is oxygen or sulfur,
R₁ and R₂ independently of one another are unsubstituted or C₁-C₄-alkoxy-, C₅-C₈-cycloalkyl-, -OH- and/or Cl-substituted C₁-C₁₈-alkyl, C₃-C₆-alkenyl, C₅-C₈-cycloalkyl, phenyl or C₇-C₉-phenylalkyl which is unsubstituted or substituted on the phenyl ring by C₁-C₄-alkyl-, C₁-C₄-alkoxy-, C₅-C₈-cycloalkyl-, -OH and/or Cl,
R₃ is H, unsubstituted or -OH-substituted C₁-C₁₈-alkyl or unsubstituted or -OH-substituted phenyl, if n is 1,
R₄ is - (C=O) -C₁-C₁₂-alkyl, -(C=O) -O-C₁-C₁₂-alkyl, -(C=O)-C₀-C₁₂-alkylene-(C=O)OZ, where Z is H or C₁-C₆-alkyl; or
R₄ is -(C=O)-phenyl, or 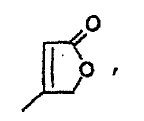 and, if
n is 2,
R₄ is the group -CHR₅-, where
R₅ is H, C₁-C₁₂-alkyl, unsubstituted or -OH-, C₁-C₄-alkyl-, C₁-C₄-alkoxy-, -C=O(OR₆) - and/or -O-COR₆-substituted phenyl and
R₆ is branched or unbranched C₁-C₁₂-alkyl or branched or unbranched C₂-C₁₂-alkenyl.

5. Composition according to Claim 4 comprising at least one epoxidized fatty acid ester.

6. Composition according to Claim 4 comprising at least one zinc carboxylate and/or alkali metal carboxylate and/or alkaline earth metal carboxylate and/or aluminium carboxylate.

7. Composition according to Claim 4 comprising at least one further substance from the groups of the phosphites, antioxidants, beta-dicarbonyl compounds, plasticizers, fillers, lubricants or pigments.

8. Composition according to Claim 4 comprising chalk as filler.

9. Composition according to Claim 4 comprising calcium stearate as lubricant.

10. Composition according to Claim 4 comprising titanium dioxide and/or zirconium oxide and/or barium sulfate as pigment.

11. Composition according to Claim 4 comprising at least one polyol and/or a disaccharide alcohol.

12. Composition according to Claim 4 comprising at least one glycidyl compound.

13. Composition according to Claim 4 comprising at least one perchlorate compound.

14. Composition according to Claim 4 comprising at least one zeolite compound.

15. Composition according to Claim 4 comprising at least one layered lattice compound.

16. Composition according to Claim 4 comprising at least one hydrotalcite.

17. Method of stabilizing of chlorine-containing polymers, **characterized in that** at least one compound of the formula II according to Claim 4 is incorporated into the polymers.

18. Use of the compounds of the general formula II according to Claim 4 as stabilizers for halogen-containing polymers and recycled halogen-containing polymers.

## Revendications

1. Composés de formule générale I 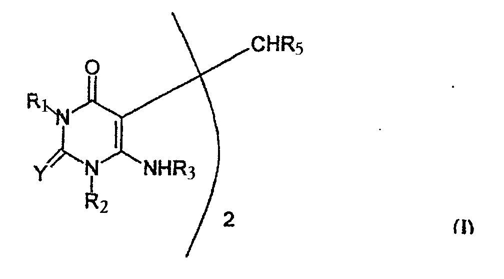 dans laquelle
Y est un oxygène ou un soufre,
R₁ et R₂ représentent, indépendamment l'un de l'autre, un alkyle en C₁ à C₁₈, un alcényle en C₃ à C₆, un cycloalkyle en C₅ à C₈, non substitué ou substitué par un alcoxy en C₁ à C₄, un cycloalkyle en C₅ à C₈, -OH et/ou Cl, un phénylalkyle en C₇ à C₉ ou un phényle, non substitué ou substitué sur le cycle phényle par un alkyle en C₁ à C₄, un alcoxy en C₁ à C₄, un cycloalkyle en C₅ à C₈, -OH et/ou Cl,
R₃ est H, un alkyle en C₁ à C₁₈ non substitué ou substitué par -OH ou un phényle non substitué ou substitué par -OH,
R₅ signifie H, un alkyle en C₁ à C₁₂, un phényle non substitué ou substitué par -OH, un alkyle en C₁ à C₄, un alcoxy en C₁ à C₄, -C=O(OR₆) et/ou -O-COR₆ et
R₆ est un alkyle en C₁ à C₁₂ ramifié ou non ramifié ou un alcényle en C₂ à C₁₂ ramifié ou non ramifié.

2. Composés de formule générale I selon la revendication 1, dans laquelle Y est un oxygène.

3. Composés de formule générale I selon la revendication 1, dans laquelle Y est un oxygène et
R₁ et R₂ représentent, indépendamment l'un de l'autre, un alkyle en C₁ à C₈, un allyle ou un phénylalkyle en C₇ à C₉.

4. Composition contenant un polymère contenant du chlore et au moins un composé de formule générale II 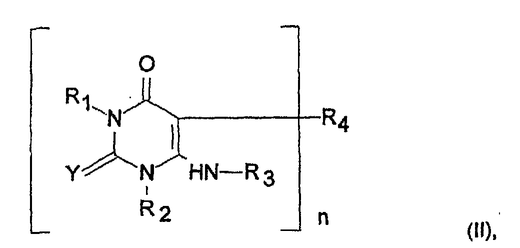 dans laquelle
n est égal à 1 ou 2,
Y est un oxygène ou un soufre,
R₁ et R₂ représentent, indépendamment l'un de l'autre, un alkyle en C₁ à C₁₈, un alcényle en C₃ à C₆, un cycloalkyle en C₅ à C₈, non substitué ou substitué par un alcoxy en C₁ à C₄, un cycloalkyle en C₅ à C₈, -OH et/ou Cl, un phénylalkyle en C₇ à C₉ ou un phényle, non substitué ou substitué sur le cycle phényle par un alkyle en C₁ à C₄, un alcoxy en C₁ à C₄, un cycloalkyle en C₅ à C₈, -OH et/ou Cl,
R₃ est H, un alkyle en C₁ à C₁₈ non substitué ou substitué par -OH ou un phényle non substitué ou substitué par -OH, lorsque n = 1,
R₄ signifie - (C=O) (alkyle en C₁ à C₁₂), - (C=O) -O- (alkyle en C₁ à C₁₂), - (C=O) (alkylène en C₀ à C₁₂) - (C=O) OZ,
Z étant H ou alkyle en C₁ à C₆, -(C=O) phényle ou 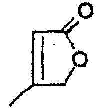 et, lorsque n = 2
R₄ signifie le groupement -CHR₅-, dans lequel
R₅ signifie H, un alkyle en C₁ à C₁₂, un phényle non substitué ou substitué par -OH, un alkyle en C₁ à C₄, un alcoxy en C₁ à C₄, -C=O(OR₆) et/ou -O-COR₆ et
R₆ est un alkyle en C₁ à C₁₂ ramifié ou non ramifié ou un alcényle en C₂ à C₁₂ ramifié ou non ramifié.

5. Composition selon la revendication 4, contenant au moins un ester époxydé d'un acide gras.

6. Composition selon la revendication 4, contenant au moins un carboxylate de zinc et/ou de métal alcalin et/ou de métal alcalino-terreux et/ou un carboxylate d'aluminium.

7. Composition selon la revendication 4, contenant au moins une autre substance des groupes des phosphites, des anti-oxydants, des composés à base de bêta-dicarbonyle, des plastifiants, des charges, des lubrifiants ou des pigments.

8. Composition selon la revendication 4, contenant de la craie comme charge.

9. Composition selon la revendication 4, contenant du stéarate de calcium comme lubrifiant.

10. Composition selon la revendication 4, contenant du dioxyde de titane et/ou de l'oxyde de zirconium et/ou du sulfate de baryum comme pigment.

11. Composition selon la revendication 4, contenant au moins un polyol et/ou au moins un alcool de disaccharide.

12. Composition selon la revendication 4, contenant au moins un composé à base de glycidyle.

13. Composition selon la revendication 4, contenant au moins un composé à base de perchlorate.

14. Composition selon la revendication 4, contenant au moins un composé zéolithique.

15. Composition selon la revendication 4, contenant au moins un composé à réseau en couches.

16. Composition selon la revendication 4, contenant au moins une hydrotalcite.

17. Procédé pour la stabilisation de polymères contenant du chlore, **caractérisé en ce qu'**on incorpore dans ceux-ci au moins un composé de formule II selon la revendication 4.

18. Utilisation des composés de formule générale II selon la revendication 4 comme stabilisateurs de polymères contenant de l'halogène et de leurs produits recyclés.
